# EUROPEAN PATENT APPLICATION

(11) **EP 4 606 439 A1**
(43) Date of publication of application: **27.08.2025**
(21) Application number: 23879738.5
(22) Date of filing: 13.10.2023
(51) Int. Cl.: A63B 69/00, A61B 5/11, A63B 71/06

(54) **INFORMATION PROCESSING METHOD, INFORMATION PROCESSING DEVICE, AND COMPUTER-READABLE NON-TRANSITORY STORAGE MEDIUM**

(30) Priority: 21.10.2022 JP 2022169309
(71) Applicant: Sony Group Corporation, Tokyo 108-0075 (JP)
(72) Inventor: ISHIMURA, Yuji, Tokyo 108-0075 (JP)
(74) Representative: D Young & Co LLP
(86) International application number: PCT/JP2023/037263
(87) International publication number: WO 2024/085095

(57) **Abstract**

An information processing method of the present disclosure includes acquiring an evaluation perspective in evaluating a motion of an evaluation target. The information processing method includes determining a configuration evaluating the motion of the evaluation target based on the evaluation perspective. The information processing method includes acquiring at least one of video information related to a motion and audio information. The information processing method includes generating content including a configuration and at least one of video information and audio information.

## Description

### Field

The present disclosure relates to an information processing method, an information processing device, and a computer-readable non-transitory storage medium.

### Background

In the field of rehabilitation, there is a proposed method of performing rehabilitation easily and appropriately using posture estimation techniques.

The posture estimation technique is a technique of extracting, from an image of a person or an object being a target, a plurality of key points (a plurality of feature points indicating a portion such as shoulder, elbow, wrist, hip, knee, and ankle, when the target is a human), and estimating the posture of the target based on relative positions of the key points. Posture estimation techniques are expected to be applied in a wide range of fields such as learning support in sports, healthcare, automated driving, and hazard prediction.

### Citation List

### Patent Literature

Patent Literature 1: JP 2020-141806 A

### Summary

### Technical Problem

It is conceivable that motion analysis using a video of the target is used for a predetermined person to train to achieve (or learn) a predetermined motion, for example. For example, the motion analysis can be used to determine whether rehabilitation is correctly performed when a specific person performs rehabilitation as a predetermined motion.

For example, in a case where the predetermined motion is a motion in rehabilitation, details of rehabilitation to be actually performed are different depending on a person who performs rehabilitation. Furthermore, the predetermined motion is not limited to motion for rehabilitation, and is assumed to include motions in various fields such as sports and exercises, for example.

For example, a predetermined person (user) performs a predetermined motion in accordance with an instruction of a correct motion, and further obtains feedback as to whether the motion has been performed correctly, enabling training and learning of the motion with higher accuracy. However, it is not easy for the user to obtain information related to the motion suitable for the user (for example, information instructing types and degrees of motion and feedback information).

In view of this, the present disclosure provides a mechanism by which a user can more easily obtain information related to the motion suitable for the user.

Note that the above problem or target is merely one of a plurality of problems or targets that can be solved or achieved by a plurality of embodiments disclosed in the present specification.

### Solution to Problem

An information processing method of the present disclosure includes acquiring an evaluation perspective in evaluating a motion of an evaluation target. The information processing method includes determining a configuration evaluating the motion of the evaluation target based on the evaluation perspective. The information processing method includes acquiring at least one of video information related to a motion and audio information. The information processing method includes generating content including a configuration and at least one of video information and audio information.

### Brief Description of Drawings

FIG. 1 is a diagram illustrating an outline of an information processing system according to an embodiment of the present disclosure.
FIG. 2 is a diagram illustrating an outline of content generation processing according to the embodiment of the present disclosure.
FIG. 3 is a block diagram illustrating a composition example of a content generation device according to the embodiment of the present disclosure.
FIG. 4 is a diagram illustrating an example of individual content generation processing according to the embodiment of the present disclosure.
FIG. 5 is a diagram for illustrating an example of a determination item definition according to the present embodiment.
FIG. 6 is a diagram illustrating an example of a user interface according to the embodiment of the present disclosure.
FIG. 7 is a diagram illustrating another example of the user interface according to the embodiment of the present disclosure.
FIG. 8 is a diagram illustrating another example of the user interface according to the embodiment of the present disclosure.
FIG. 9 is a diagram illustrating another example of the user interface according to the embodiment of the present disclosure.
FIG. 10 is a diagram illustrating another example of the user interface according to the embodiment of the present disclosure.
FIG. 11 is a diagram illustrating a list example of sites according to the embodiment of the present disclosure.
FIG. 12 is a diagram illustrating another example of the user interface according to the embodiment of the present disclosure.
FIG. 13 is a view illustrating a list example of joint parts according to the embodiment of the present disclosure.
FIG. 14 is a diagram illustrating another example of the user interface according to the embodiment of the present disclosure.
FIG. 15 is a diagram illustrating another example of the user interface according to the embodiment of the present disclosure.
FIG. 16 is a view illustrating a list example of site parts according to the embodiment of the present disclosure.
FIG. 17 is a diagram illustrating another example of the user interface according to the embodiment of the present disclosure.
FIG. 18 is a diagram illustrating another example of the user interface according to the embodiment of the present disclosure.
FIG. 19 is a diagram illustrating an example of a verification result according to the embodiment of the present disclosure.
FIG. 20 is a diagram illustrating another example of the verification result according to the embodiment of the present disclosure.
FIG. 21 is a diagram illustrating an example of individual content according to the embodiment of the present disclosure.
FIG. 22 is a block diagram illustrating a composition example of a content generation device according to the embodiment of the present disclosure.
FIG. 23 is a diagram illustrating another example of individual content generation processing according to the embodiment of the present disclosure.
FIG. 24 is a block diagram illustrating a composition example of a content providing device according to the embodiment of the present disclosure.
FIG. 25 is a diagram illustrating an example of a user interface of a prescription application according to the embodiment of the present disclosure.
FIG. 26 is a diagram illustrating an example of a user interface of a distribution application according to the embodiment of the present disclosure.
FIG. 27 is a diagram illustrating an example of an application installation method according to the embodiment of the present disclosure.
FIG. 28 is a diagram illustrating another example of the application installation method according to the embodiment of the present disclosure.
FIG. 29 is a hardware composition diagram illustrating an example of a computer that implements functions of the content generation device according to the present disclosure.

### Description of Embodiments

Hereinafter, embodiments of the present disclosure will be described in detail with reference to the accompanying drawings. In the present specification and the drawings, substantially the same elements are denoted by the same reference numerals, and redundant description will be omitted.

Furthermore, specific values, which might be used in the present specification and the drawings, are merely examples, and other values may be applied.

One or a plurality of embodiments (implementation examples and modifications) described below can each be implemented independently. On the other hand, at least some of the plurality of embodiments described below may be appropriately combined with at least some of other embodiments. The plurality of embodiments may include novel features different from each other. Accordingly, the plurality of embodiments can contribute to achieving or solving different objects or problems, and can exhibit different effects.

### <<1. Outline of information processing system>>

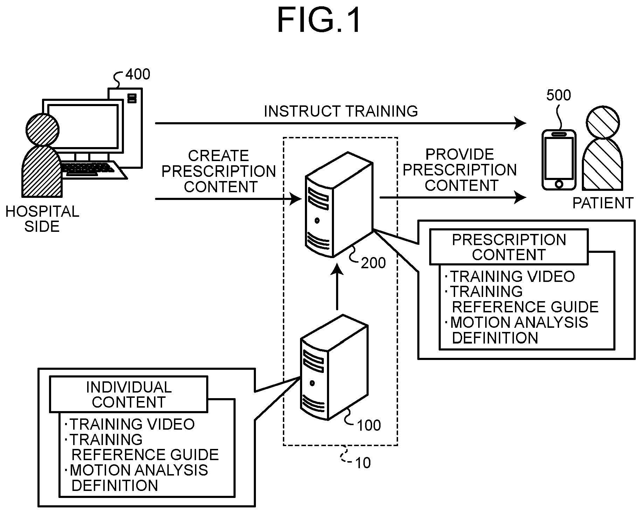

FIG. 1 is a diagram illustrating an outline of an information processing system 10 according to an embodiment of the present disclosure. Here, the information processing system 10 according to the present embodiment is assumed to be a system that provides a rehabilitation assistance service, but as will be described below, the service provided by the information processing system 10 is not limited to rehabilitation assistance.

For example, the information processing system 10 assists rehabilitation provision (hereinafter, also referred to as provision assistance) in which a medical institution such as a hospital instructs a patient to perform rehabilitation. Furthermore, the information processing system 10 provides assistance (hereinafter, also referred to as execution assistance) when a patient performs rehabilitation in accordance with an instruction from a medical institution.

"Rehabilitation" refers to techniques and methods for enhancing the potential of a patient subjected to an extended treatment period, such as a patient with disorders, chronic diseases, and geriatric diseases, to restore and promote vital functions, and thus social functions. In addition, a person who is a target of rehabilitation is denoted as a patient. Examples of the patient include a person with illness of injury, an elderly person, and a person with disabilities.

For example, medical persons such as doctors, physical therapists, pharmacists, and health advisers on the hospital side determines a training menu for rehabilitation in consideration of conditions of the patient, and instruct the patient to perform the determined training menu. At a place such as a home, the patient performs the training menu determined by the hospital side.

At this time, for example, conveying training menus verbally or on paper to the patient by a medical person would make it difficult for the patient to correctly perform the training. When the training instruction is conveyed verbally or on paper, the patient needs to perform the training at places such as a home while remembering the instruction. In this manner, the amount of information that can be conveyed to the patient verbally or on paper is small, increasing the burden on the patient who actually performs the training. In addition, when training is performed at places such as home, there is a problem of difficulty for the patient to objectively grasp their own motion, making it difficult to confirm whether the training is correctly performed.

In view of these, a content providing device 200 of the information processing system 10 of the present embodiment provides prescription content, including a training video, a training reference guide, and a motion analysis definition, to a patient in accordance with a creation instruction from a medical person on the hospital side.

The content providing device 200 receives a creation instruction from a medical person on the hospital side via an information processing device 400 used by the medical person. Having received the creation instruction, the content providing device 200 generates prescription content and provides the generated prescription content to an information processing device 500 used by the patient. Once having received the creation instruction from the information processing device 500, the content providing device 200 can thereafter provide the prescription content to the information processing device 400 without receiving the creation instruction. For example, having acquired identification information of specific content from the information processing device 500, the content providing device 200 can provide prescription content corresponding to the identification information to the information processing device 500.

With this method, the patient can execute training for rehabilitation based on the training video and the training reference guide.

In addition, the prescription content includes a motion analysis definition. Therefore, by capturing the video of the currently performed training (hereinafter, also referred to as a training execution video), the patient can confirm an analysis result of the training execution video. This allows the patient to perform training with higher accuracy.

Here, generating the prescription content by a medical person on the hospital side from scratch would increase the burden on the medical person. This is because the training menu to be performed and the definition of the motion analysis vary depending on the conditions of the patient. Therefore, creating the prescription content from scratch for each training or for each patient would increase the burden on the medical person.

In particular, performing a motion analysis of the training execution video automatically by the information processing system 10 will require a motion analysis definition in a format that can be processed by the information processing system 10. On the other hand, the motion analysis of rehabilitation requires medical knowledge. In this manner, generation of the motion analysis definition requires knowledge related to the information processing system 10 in addition to medical knowledge. This has made it difficult for a medical person to generate the motion analysis definition.

In view of this, in the information processing system 10 of the present embodiment, the content generation device 100 generates, in advance, individual content including the training video, the training reference guide, and the motion analysis definition. The medical person combines the individual content or modifies (edits) the individual content to create prescription content appropriate for the patient. Alternatively, the medical person can generate individual content and generate prescription content including the generated individual content. In this case, as described below, the content generation device 100 provides a medical person with a tool (UI) for generating individual content.

In this manner, the medical person generates the prescription content including at least one of the individual content generated in advance by the content generation device 100, the content obtained by modifying the individual content, and the individual content generated by the medical person themselves. The prescription content includes one or more pieces of individual content. When the prescription content includes one piece of individual content, this individual content is to be the prescription content. That is, the individual content itself can be provided to the patient as prescription content.

As described below, an information processing device 300 may include the functions of the content generation device 100, or the content generation device 100 need not be included in the information processing system 10.

This allows the medical person to more easily create the prescription content appropriate for the patient even when the medical person has less knowledge about the information processing system 10.

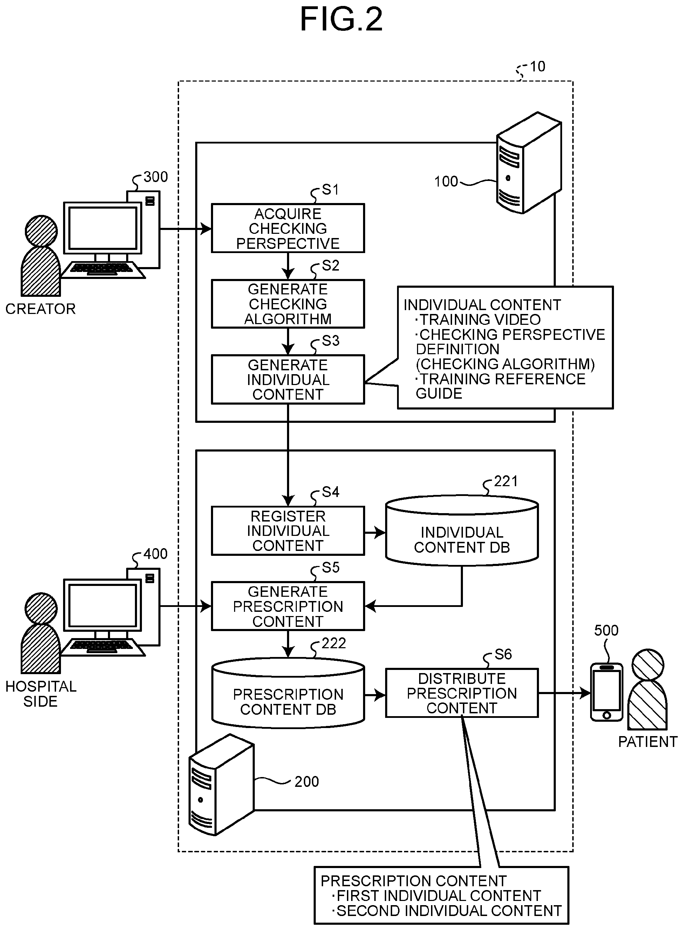

Next, an example of content generation processing including generating individual content and distributing prescription content will be described with reference to FIG. 2.

FIG. 2 is a diagram illustrating an outline of content generation processing according to the embodiment of the present disclosure. The content generation processing illustrated in FIG. 2 is executed by the information processing system 10. The information processing system 10 includes the content generation device 100 and the content providing device 200. The content providing device 200 includes: an individual content database (DB) 221 that stores individual content; and a prescription content DB 222 that stores prescription content.

First, the content generation device 100 of the information processing system 10 acquires a training checking perspective (an example of an evaluation perspective) from the creator of the individual content via the information processing device 300 (step S1). The checking perspective is information related to an evaluation method including a timing of analyzing the motion included in the training, and evaluation items, for example, "Right arm is horizontal to ground when right hand is extended forward". The creator selects one or more checking perspectives from a plurality of checking perspectives held by the content generation device in preparation, the content generation device 100 acquires the checking perspectives from the creator. Alternatively, the creator may generate the checking perspectives. In this case, the content generation device 100 acquires the checking perspectives generated by the creator.

Next, the content generation device 100 generates a checking algorithm (an example of a configuration) of evaluating a motion based on the acquired checking perspective (step S2). The checking algorithm is an example of the motion analysis definition described above, for example.

The content generation device 100 generates individual content including the generated checking algorithm (step S3), and outputs the generated individual content to the content providing device 200. In addition to the checking algorithm (checking perspective definition), the individual content can include, for example, a training video (an example of video information related to motion) and a training reference guide, acquired from the creator.

In this manner, the content generation device 100 generates the checking algorithm based on the checking perspective acquired from the creator. With this method, the creator can more easily generate the checking algorithm (motion analysis definition).

Furthermore, for example, the content generation device 100 can provide the creator with a tool (UI) enabling the creator to generate the checking perspective more intuitively. For example, the content generation device 100 provides the creator with a UI enabling easier input of information related to checking standard, for example, which site of the human body is to be checked or at which timing the check is to be performed. Details of the UI will be described below. Furthermore, the content generation device 100 can provide the creator with a componentized product of the checking perspective and the checking algorithm that have already been generated, for example. With this method, the creator can more easily create the checking algorithm regardless of the presence or absence of programming knowledge (for example, programming language comprehension and programming skills).

In addition to or in place of the training video, training audio (an example of audio information) instructing a training method may be included in the individual content. This allows the patient to perform the training in accordance with the audio.

For example, there is a case where it is difficult for the patient to capture the video of the state of the training while performing the training by viewing the training video. In this case, by using the training audio instead of the training video, the patient can feel easier when capturing the video of the state of the training while performing the training.

Having acquired the individual content from the content generation device 100, the content providing device 200 in FIG. 2 registers the acquired individual content in the individual content DB 221 (step S4).

Next, the content providing device 200 generates prescription content appropriate for a patient in accordance with an instruction from a medical person on the hospital side (step S5), and registers the prescription content in the prescription content DB 222. For example, the content providing device 200 generates prescription content including at least one piece of individual content registered in the individual content DB 221. At this time, the content providing device 200 may generate the prescription content by changing a part of the individual content.

The content providing device 200 distributes the prescription content registered in the prescription content DB 222 to the patient (step S6). The prescription content distributed to the patient includes various types of individual content (in the example of FIG. 2, first individual content and second individual content), appropriate for the patient.

In this manner, the content providing device 200 generates the prescription content based on the individual content, making possible for the medical person on the hospital side to more easily create the prescription content. In addition, the patient can acquire the prescription content more easily.

Here, the creator who creates the individual content is different from the medical person on the hospital side who creates the prescription content, but the creator and the medical person may be the same person. That is, the medical person on the hospital side can create the individual content by themselves and create the prescription content including the created individual content.

In addition, in the present embodiment, in order to simplify the description, the individual content and the prescription content are assumed to be content for the patient to perform rehabilitation training, but the individual content and the prescription content are not limited to this example.

The individual content and the prescription content may be any content used by the user to learn a motion. For example, the individual content and the prescription content may be content for performing training such as exercise, sports, fitness, and dance. Alternatively, for example, the individual content and the prescription content may be content for learning techniques such as traditional performing arts and product manufacturing. In the case of the rehabilitation described above, the patient corresponds to a user who learns a motion. In the case of exercise, sports, fitness, dance, and the like, a person who performs these activities corresponds to a user who learns a motion. In addition, in a case of acquiring a technique such as a traditional art, production of a product, or the like, a person who acquires the technique corresponds to a user who acquires a motion.

Examples of the information processing device 300 used by the creator, the information processing device 400 used by a medical person on the hospital side, and the information processing device 500 used by the patient include devices such as a personal computer (PC), a tablet, and a smartphone.

### <<2. Content generation device>>

Next, the content generation device 100 will be described. The content generation device 100 is an information processing device that generates individual content. The content generation device 100 may be an edge computer that performs edge processing near the user, or may be a cloud computer that performs processing on the cloud side, that is, may be a server device.

### <2.1. Composition example of content generation device>

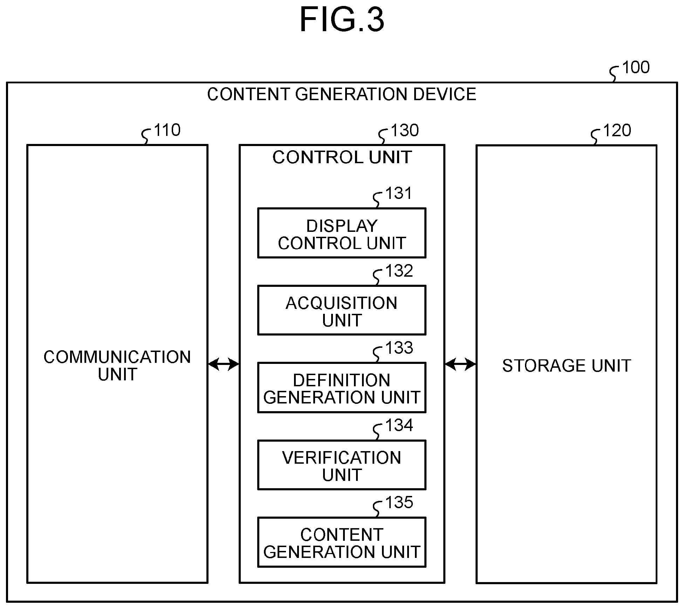

FIG. 3 is a block diagram illustrating a composition example of the content generation device 100 according to the embodiment of the present disclosure. The content generation device 100 includes a communication unit 110, a storage unit 120, and a control unit 130. Note that the composition illustrated in FIG. 3 is a functional composition, and the hardware composition may be different from this. Furthermore, the functions of the content generation device 100 may be implemented in a distributed manner in a plurality of physically separated structures. For example, the content generation device 100 may be constituted with a plurality of server devices.

### [Communication unit 110]

The communication unit 110 is a communication interface for communicating with other devices. The communication unit 110 may be a network interface or a device connection interface. For example, the communication unit 110 may be a local area network (LAN) interface such as a network interface card (NIC), or may be a universal serial bus (USB) interface including a USB host controller, a USB port, and the like. Furthermore, the communication unit 110 may be a wired interface, or may be a wireless interface.

The communication unit 110 functions as a communication means in the content generation device 100. The communication unit 110 communicates with the content providing device 200 and the information processing device 300 used by the creator.

### [Storage unit 120]

The storage unit 120 is a data readable/writable storage device such as dynamic random access memory (DRAM), static random access memory (SRAM), a flash drive, or a hard disk. The storage unit 120 functions as a storage means in the content generation device 100.

### [Control unit 130]

The control unit 130 is actualized by execution of various programs stored in a storage device inside the content generation device 100 by a central processing unit (CPU), a micro processing unit (MPU), or the like, using the RAM as a work area. Furthermore, the control unit 130 is actualized by, for example, an integrated circuit such as an application specific integrated circuit (ASIC) or a field programmable gate array (FPGA).

As illustrated in FIG. 3, the control unit 130 includes a display control unit 131, an acquisition unit 132, a definition generation unit 133, a verification unit 134, and a content generation unit 135, and achieves or executes a function and an action of information processing described below. The internal composition of the control unit 130 is not limited to the composition illustrated in FIG. 3, and may be another composition as long as it is a composition that performs information processing described below.

### (Display control unit 131)

The display control unit 131 generates a screen to be presented to the creator, for example, and controls the information processing device 300 to display the created screen used by the creator. For example, the display control unit 131 notifies the information processing device 300 to display the generated screen information, thereby notifying the creator of screen information.

### (Acquisition unit 132)

The acquisition unit 132 acquires various types of information from the creator via the information processing device 300. For example, the creator inputs various types of information to the information processing device 300 in accordance with the screen information. The information processing device 300 notifies the content generation device 100 of the various types of information input by the creator. The acquisition unit 132 acquires various types of information based on the notification from the information processing device 300.

The acquisition unit 132 acquires, for example, training information such as information related to checking perspectives, training video information, training audio information, and a reference guide related to training. as various types of information. The acquisition unit 132 outputs the acquired information to the definition generation unit 133, the verification unit 134, and the content generation unit 135.

### (Definition generation unit 133)

The definition generation unit 133 generates a motion analysis definition (checking algorithm) based on the information related to the checking perspectives acquired by the acquisition unit 132. The definition generation unit 133 outputs the generated motion analysis definition to the verification unit 134 and the content generation unit 135.

In addition, the definition generation unit 133 may update the motion analysis definition using information input by the creator based on the verification result obtained by the verification unit 134. The definition generation unit 133 outputs the updated motion analysis definition to the verification unit 134 and the content generation unit 135.

### (Verification unit 134)

Based on the motion analysis definition generated by the definition generation unit 133, the verification unit 134 analyzes the training video information acquired by the acquisition unit 132 and generates an analysis result. For example, the verification unit 134 generates the analysis result by executing posture analysis on the training video information by using a posture analysis algorithm, and by performing motion analysis defined in the motion analysis definition by using a result of the posture analysis. The verification unit 134 instructs the display control unit 131 to present the analysis result to the creator, or example.

Note that the analysis of the training video information may be performed by the verification unit 134, or may be performed by an external device (for example, an analysis device (not illustrated)). That is, the information processing system 10 may have the analysis function of analyzing the training video information based on the motion analysis definition installed in the content generation device 100 or installed in an external analysis device. In a case where the analysis function is installed in an external analysis device, the analysis device may be a device included in the information processing system 10 or a device outside the information processing system 10. This analysis function can also be used for motion analysis of a video of training states captured by the patient themselves (training execution video) by a prescription application to be described below, for example.

### (Content generation unit 135)

The content generation unit 135 generates individual content including the information acquired by the acquisition unit 132 and the motion analysis definition generated by the definition generation unit 133. The content generation unit 135 outputs the generated individual content to the content providing device 200 via the communication unit 110.

### <2.2. Individual content generation processing>

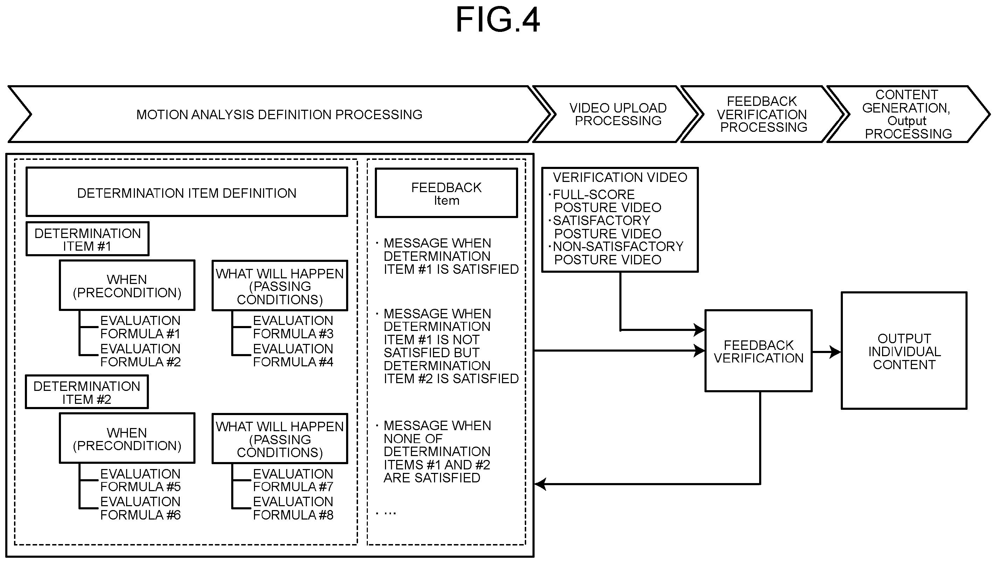

FIG. 4 is a diagram illustrating an example of individual content generation processing according to the embodiment of the present disclosure. As illustrated in FIG. 4, the individual content generation processing includes motion analysis definition processing, video upload processing, feedback verification processing, the content generation, and output processing.

### [Motion analysis definition processing]

The motion analysis definition processing is processing of acquiring a checking perspective from the creator and determining a configuration (motion analysis definition), being a definition for evaluating the motion of an evaluation target person based on the acquired checking perspective.

The checking perspective includes information related to a determination item (an example of determination item definition and determination information) and information related to feedback (an example of feedback Item and evaluation information).

### (Determination item definition)

The determination item definition includes one or more determination items. In the example of FIG. 4, the determination item definition includes two determination items (determination items #1 and #2). The determination item includes: a precondition indicating when to perform determination (evaluation); and a passing condition indicating what condition leads to what evaluation. The precondition and the passing condition include one or more evaluation formulas. The evaluation formula is expressed, for example, in the form of logical operation.

For example, in FIG. 4, evaluation formulas #1 and #2 are defined as preconditions of the determination item #1. Evaluation formulas #3 and #4 are defined as passing conditions of the determination item #1.

The number of determination items included in the determination item definition is not limited to two, and may be one, or three or more. In addition, the number of evaluation formulas included in the precondition is not limited to two, and may be one, or three or more. The number of evaluation formulas included in the passing condition is not limited to two, and may be one, or three or more.

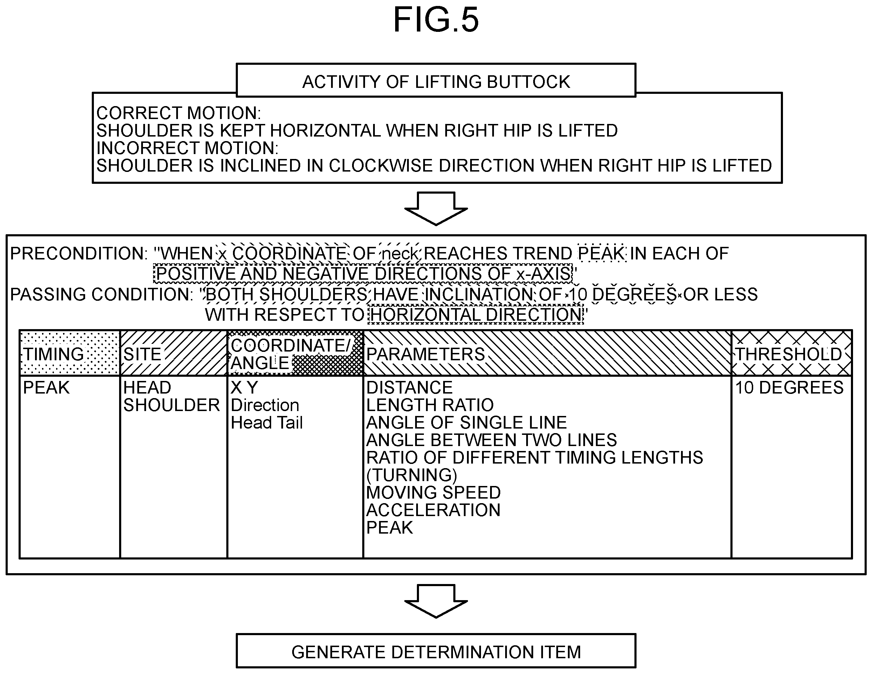

FIG. 5 is a diagram for illustrating an example of a determination item definition according to the present embodiment. Here, a case of performing an exercise of lifting the buttocks as training will be described. In this training, the motion of the patient is evaluated as a good motion when the shoulder (line segment connecting both shoulders) is kept horizontal at the time of lifting the right hip, and is evaluated as a bad motion when the shoulder is inclined clockwise or counterclockwise at the time of lifting the right hip.

In this case, the content generation device 100 acquires as the checking perspective, information of "When the x-coordinate of neck reaches a trend peak in each of the positive and negative directions of the x-axis, both shoulders have an inclination of 10 degrees or less with respect to the horizontal direction" from the creator. Here, the inclination of the shoulders with respect to the horizontal direction is an angle formed by a line segment connecting the shoulders and a horizontal line. The content generation device 100 acquires information that the angle is 10 degrees or less in the clockwise direction or the counterclockwise direction.

Here, the information "When the x-coordinate of neck reaches a trend peak in each of the positive and negative directions of the x-axis" corresponds to a precondition, that is, a timing to perform evaluation. In addition, information that "both shoulders have an inclination of 10 degrees or less with respect to the horizontal direction" corresponds to the passing condition, that is, the condition to be evaluated.

Here, the content generation device 100 acquires the information that the angle formed by the horizontal direction of both shoulders is 10 degrees or less in the clockwise direction or the counterclockwise direction as the checking perspective, but the information acquired by the content generation device 100 is not limited to this information. For example, the content generation device 100 may separately acquire information that the angle formed in the clockwise direction is 10 degrees or less and information that the angle formed in the counterclockwise direction is 10 degrees or less. The motion of the body varies depending on whether the line connecting the both shoulders is inclined in the clockwise direction or the counterclockwise direction. Accordingly, the content generation device 100 can acquire the checking perspective separately in each of the clockwise direction and the counterclockwise direction.

The content generation device 100 extracts information necessary for generating the configuration from the acquired checking perspective. For example, the content generation device 100 extracts element information related to timing, a site, coordinates/angles, parameters, and thresholds, from the checking perspective.

Here, the precondition is a timing at which the evaluation is performed, that is, a timing to extract and analyze the motion. Examples of the precondition include a peak time illustrated in FIG. 5, a predetermined angle, distance, time, and the like. The predetermined angle is, for example, a timing at which a predetermined site such as an arm is open to a predetermined angle. The predetermined distance is, for example, timing at which a predetermined site such as a hand is away from a body or the like by a predetermined distance. In addition, the predetermined time is, for example, a timing at which a predetermined time has elapsed after a predetermined site such as a hand starts moving. Furthermore, in addition to the preconditions described above, the following period can also be defined as the precondition: a moment at which a specific condition is satisfied (including a peak time); a period defined by a moment and another moment at which a specific condition is satisfied; an entire period of a video to be analyzed; and a period (moment) to be analyzed within this video. Examples of the moment at which the specific condition is satisfied include a moment at which the angle between the horizontal direction and the line segment connecting the shoulders changes from 60 degrees or less to 60 degrees or more.

The content generation device 100 extracts the information related to the timing from the checking perspective.

The site is a site to be evaluated, as an evaluation target. The information related to the site includes, for example, information related to a site of the body of an evaluation target person who receives evaluation of motion, such as a patient. Examples of the information related to the site include information related to the head and the shoulder. For example, in the case of the above-described motion of lifting the buttock, the content generation device 100 extracts "neck" and "shoulders" as sites.

The coordinates and angles are coordinates and angles to be evaluated. The information related to coordinates and angles includes: for example, coordinates as an evaluation target, such as the x axis and the y axis; and information related to a direction as evaluation target, such as an x axis direction and a y axis direction. The information related to coordinates and angles may be expressed as a vector. In the case of the motion of lifting the buttock described above, the content generation device 100 extracts "positive and negative directions on x-axis" or "horizontal direction" as the coordinate/angle.

The parameter is a parameter used for evaluation. The information related to parameter includes information related to a ratio of a distance or ratio of a length, an angle of a single line (an angle with respect to a coordinate axis), an angle between two lines, a ratio of lengths of different timings (turning), a moving speed, acceleration, a peak, and the like. For example, in the case of the above-described motion of lifting the buttock, the content generation device 100 extracts "x coordinate" and "inclination" as parameters.

The threshold is a value used to evaluate the parameter. The information related to threshold may be extracted for each parameter, for example. For example, in the case of the above-described motion of lifting the buttock, the content generation device 100 extracts "10 degrees" as the threshold.

The content generation device 100 expresses the extracted element information with a logical operation formula to generate a determination item each including the evaluation formula of the precondition and the evaluation formula of the passing condition.

### (Feedback Item)

Returning to FIG. 4, the content generation device 100 extracts a feedback Item from the checking perspective. The feedback Item represents, for example, information (for example, a message) returned, as feedback, to the user (patient) who is the evaluation target person when the passing condition is satisfied or not satisfied.

The content generation device 100 generates the feedback Item in accordance with an input from the creator, for example. The content generation device 100 generates a message corresponding to whether the passing condition of the determination item is satisfied, as the feedback Item. In a case where the determination item exists in plurality, the content generation device 100 generates a message corresponding to a combination of the determination items as the feedback Item.

For example, in FIG. 4, the content generation device 100 generates, as the feedback Item, message according to each combination of determination items, such as a message when the determination item #1 is satisfied and a message when the determination item #1 is not satisfied but the determination item #2 is satisfied. Examples of the feedback Item include information such as character information of a message, information for converting a message into spoken audio by a Text-To-Speech function (TTS), information related to a video or audio corresponding to a message, and information indicating a position of these pieces of information (for example, URL).

### (Example of interface for acquiring check definition)

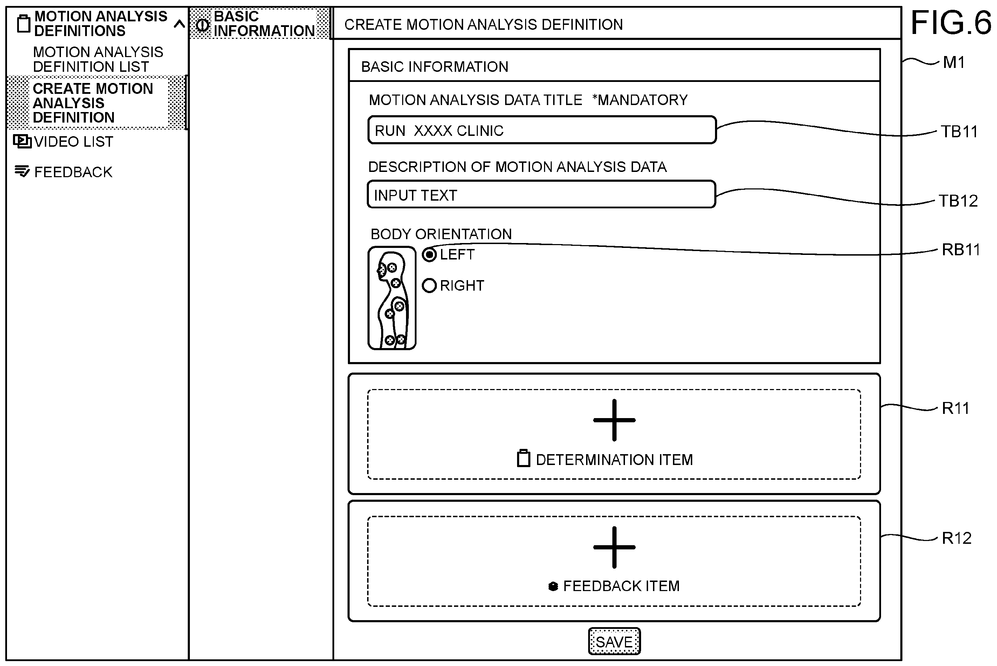

An example of a user interface presented to the creator by executing the motion analysis definition processing will be described with reference to FIGS. 6 to 18.

Hereinafter, when the motion analysis definition processing is under execution, the presentation of information to the information processing device 300 used by the creator is supposed to be executed by the display control unit 131, and the acquisition of information by operation (input) on the information displayed on the information processing device 300 is supposed to be performed by the acquisition unit 132.

FIG. 6 is a diagram illustrating an example of a user interface according to the embodiment of the present disclosure. FIG. 6 illustrates a first screen M1 presented to the creator, for example, when the creator generates a new motion analysis definition.

As illustrated in FIG. 6, on the first screen M1, the content generation device 100 receives, at a text box TB11, an input of a title (motion analysis data title) of a motion analysis definition to be newly created. In addition, the content generation device 100 receives, at a text box TB12, an input of description of the motion analysis definition (motion analysis data).

In addition, the content generation device 100 includes a radio button RB11 to receive selection of the body orientation of the evaluation target person to be used when the creator performs motion analysis definition, that is, inputs the checking perspective.

In FIG. 6, either the left or the right can be selected as the orientation of the body of the evaluation target person, but the orientation of the body is not limited thereto. The orientation of the body of the evaluation target person that can be selected may include front and back, for example. Furthermore, here is a case where the body of the evaluation target person is displayed in 2D is illustrated, but the body of the evaluation target person may be displayed in 3D. In this case, the creator may select (or switch) 2D or 3D.

In addition, the first screen M1 includes: a determination item region R11 to receive an input of the determination item; and a feedback item region R12 to receive an input of the feedback Item.

The creator can click on the determination item region R11 and the feedback item region R12 to respectively input a determination item and a feedback Item from scratch. Alternatively, the creator may generate a new motion analysis definition using a previously created motion analysis definition or a motion analysis definition created by another person.

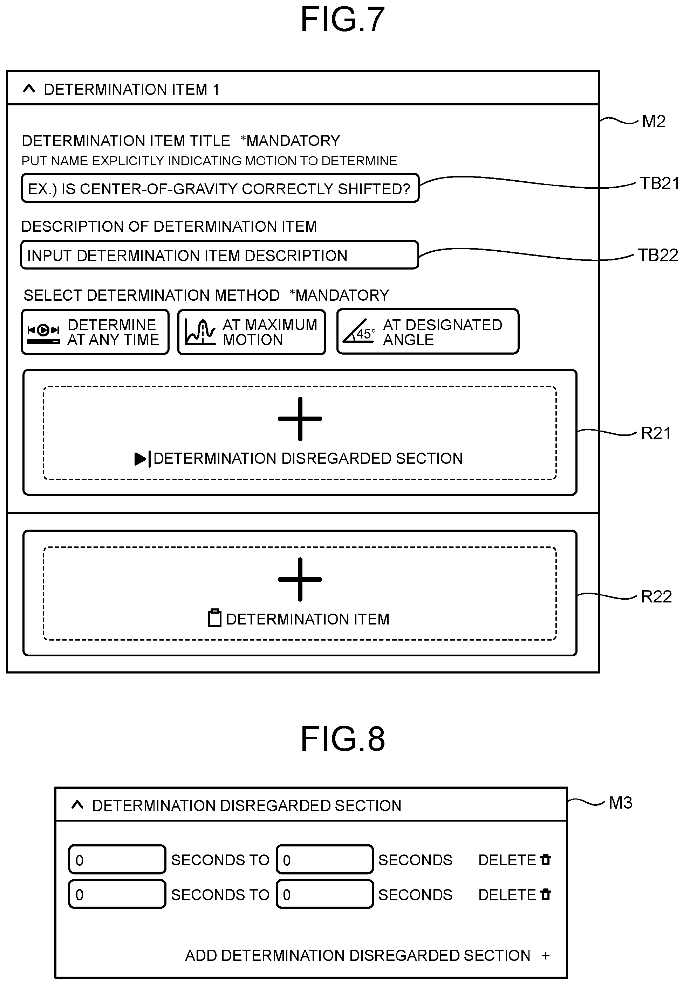

FIG. 7 is a diagram illustrating another example of the user interface according to the embodiment of the present disclosure. FIG. 7 illustrates, for example, a second screen M2 presented to the creator when the determination item region R11 is selected (for example, by clicking). The second screen M2 may be displayed by transitioning from the first screen M1, or the second screen M2 may be displayed on a part of the first screen M1 or superimposed on the first screen M1.

The content generation device 100 receives an input of one or more checking perspectives regarding one motion posture via the second screen M2. For example, the content generation device 100 receives, via the second screen M2, an input of a determination item for checking whether the center-of-gravity shift is correctly performed in the case of the rising motion. Alternatively, the content generation device 100 receives, via the second screen M2, an input of a determination item for checking whether the timing of releasing the ball or the height of the elbow is correct at the time of a pitching motion in baseball.

As illustrated in FIG. 7, on the second screen M2, the content generation device 100 receives an input of a title (determination item title) of a determination item to be newly created, at a text box TB21. In addition, the content generation device 100 receives, at a text box TB22, an input of description of the determination item.

In addition, the content generation device 100 receives the selection of the determination method. The content generation device 100 receives, as a determination method, timing of performing determination (precondition), for example, "make determination at any time", "(make determination) at maximum motion", or "(make determination) at a designated angle". As described above, the determination method includes a case where the determination is made at any time and a case where the determination is performed using a certain condition such as "at maximum motion" or "at a designated angle" as a trigger.

The determination method is not limited to the example described above. For example, the determination method may be a method of designating a specific time, distance, or the like, such as "designated time".

In addition, the creator can set a section in which no determination is made (determination disregarded section). For example, the creator clicks a determination disregarded section region R21 to set a section in which no determination is made.

For example, in many cases, the training is not started for a predetermined period after the training video or the training audio starts playing. In this manner, by starting the training after a certain period from the start of play of the training video or the training audio, the patient can start the training without feeling pressured to hurry.

In this manner, by setting the section in which training is known not to be performed in advance as the determination disregarded section, it is possible to decrease burden of the motion analysis processing.

FIG. 8 is a diagram illustrating another example of the user interface according to the embodiment of the present disclosure. FIG. 8 illustrates, for example, a third screen M3 presented to the creator when the determination disregarded section region R21 is selected (for example, by clicking). The third screen M3 may be displayed by transitioning from the second screen M2, or the third screen M3 may be displayed on a part of the second screen M2 or superimposed on the second screen M2.

As illustrated in FIG. 8, the creator inputs the start time and the end time of the determination disregarded section to set the determination disregarded section. The creator can also set a plurality of determination disregarded sections.

Returning to FIG. 7, the creator selects a determination method and clicks the determination item region R22 to input a determination condition (passing condition) of the determination item.

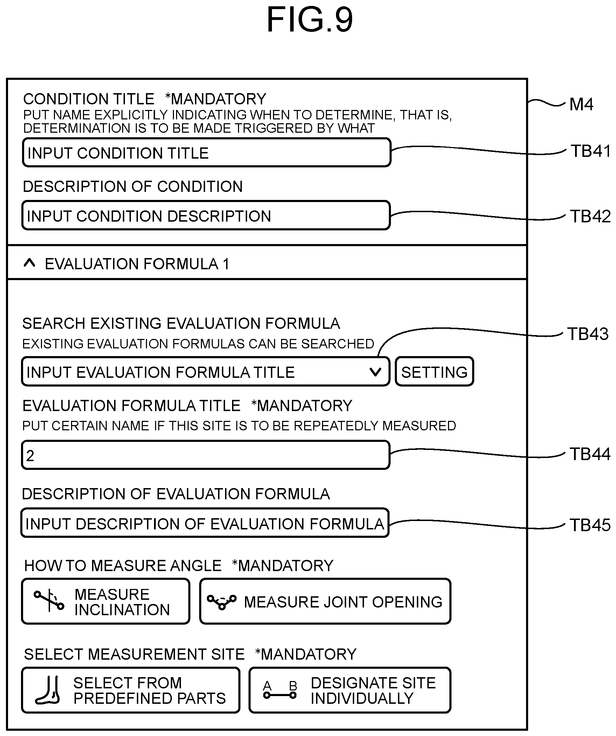

FIG. 9 is a diagram illustrating another example of the user interface according to the embodiment of the present disclosure. FIG. 9 illustrates a fourth screen M4 presented to the creator when the determination item region R22 is selected in a state where, for example, "determination is made at any time" is selected as the determination method. The fourth screen M4 may be displayed by transitioning from the second screen M2, or the fourth screen M4 may be displayed on a part of the second screen M2 or superimposed on the second screen M2.

On the fourth screen M4, the content generation device 100 receives the input of the evaluation formula of the determination item.

As illustrated in FIG. 9, on the fourth screen M4, the content generation device 100 receives an input of a title (determination item title) of a determination item to be newly created, at a text box TB41. In addition, the content generation device 100 receives an input of description of the condition, at a text box TB42.

The creator can generate a new evaluation formula using the evaluation formula previously created or the evaluation formula created by another person. For example, inputting the evaluation formula title in the text box TB43 enables the creator to use the existing evaluation formula as a template. Alternatively, the creator may select an existing evaluation formula from a pull-down menu.

The template here is a template of a previously-created evaluation formula or an evaluation formula created by another person, and can be predefined for each motion of the evaluation target or for each training details such as rehabilitation or exercise, for example. Alternatively, the template may be predefined for each evaluation target such as race, gender, and age. Furthermore, the template may be predefined for each target field of training, such as a medical field or an item of sports, or may be predefined for each country or locational region.

In addition, on the fourth screen M4, the content generation device 100 receives an input of a title (evaluation formula title) of an evaluation formula to be newly created, at a text box TB44. In addition, the content generation device 100 receives an input of description of the evaluation formula in a text box TB45.

The creator selects a method of measuring an angle to be evaluated and a method of selecting a measurement site, and inputs evaluation conditions. This corresponds to the input of the passing condition. For example, the creator selects whether to determine the inclination or measure the opening of the joint, as a method of measuring the angle. In addition, the creator selects, as a method of selecting a measurement site, whether to select from predefined sites or individually designate sites.

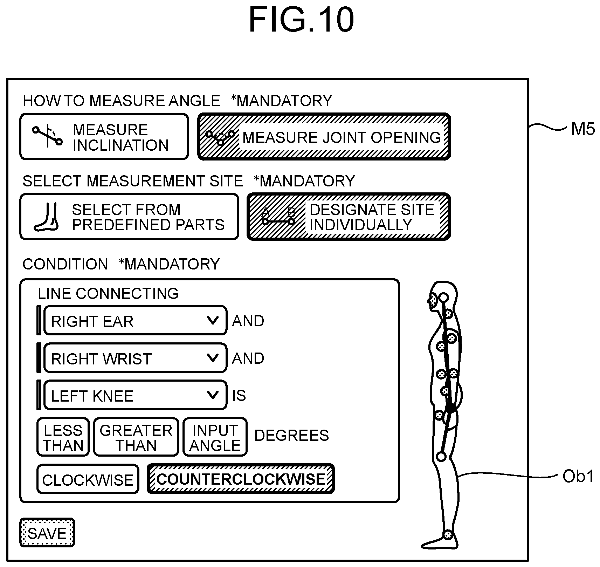

FIG. 10 is a diagram illustrating another example of the user interface according to the embodiment of the present disclosure. FIG. 10 illustrates a fifth screen M5 presented to the creator, for example, when "measure the opening of the joint" is selected as the method of measuring the angle and "individually designate site" is selected as the selection of the measurement site.

As illustrated in FIG. 10, the content generation device 100 receives designation of a site to be evaluated. In this case, the content generation device 100 receives designation of three sites in order to measure the opening of the joint.

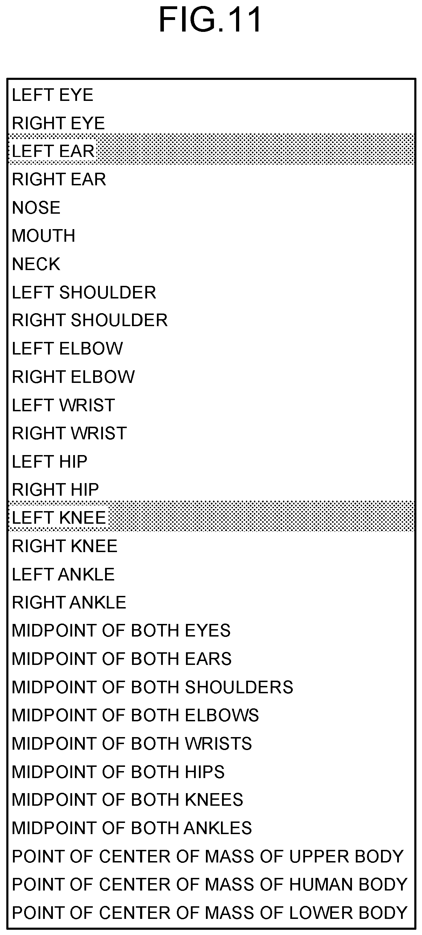

FIG. 11 is a diagram illustrating a list example of sites according to the embodiment of the present disclosure. The site is designated, for example, by being selected by the creator from a pull-down menu displaying a list of sites as illustrated in FIG. 11.

As illustrated in FIG. 11, the site that can be designated by the creator includes not only sites of the human body such as the nose and the right ankle, for example, but also positions calculated from the sites of the human body, such as a midpoint of the hips and a point of center of mass of a human body.

When the creator selects a site from the pull-down menu in FIG. 11, the selected site is highlighted on a human object Ob1 indicating the human body as illustrated in FIG. 10. With this method, the creator can easily perform visual confirmation as to which site has been selected.

The selection of the site is not limited to the method using the pull-down menu. For example, the creator may select a site displayed on the human object Ob1 (a position indicated by a circle of the human object Ob1 in FIG. 10) to select the site to be the measurement target.

In addition, on the fifth screen M5, the creator can designate a joint opening direction. In the example of FIG. 10, the creator designates whether the joint opening direction is clockwise or counterclockwise.

On the fifth screen M5, the creator inputs a threshold of the passing condition. Here, the creator designates the angle of the joint, and designates whether the condition is to be satisfied when the angle of the joint is less than the designated angle or the condition is to be satisfied when the angle of the joint is greater than the designated angle.

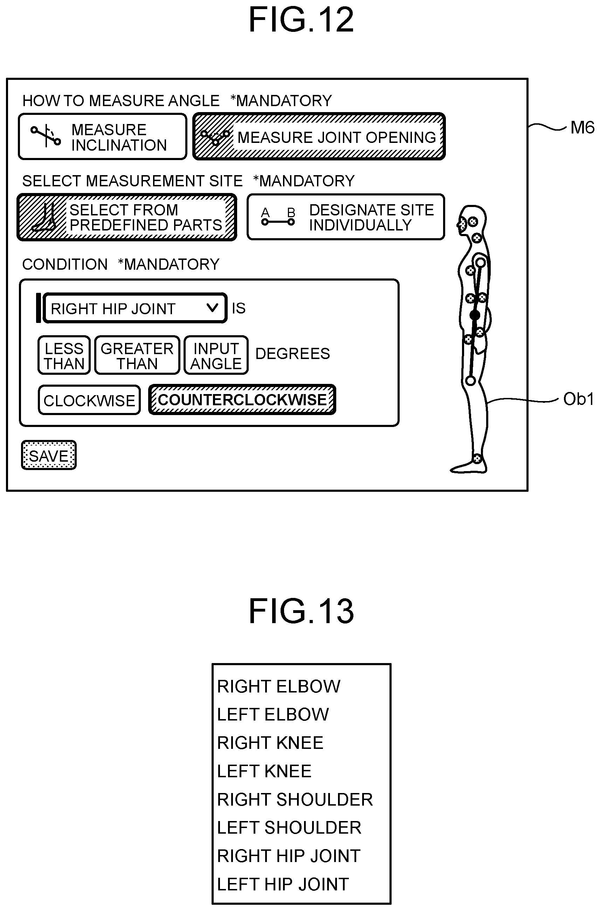

FIG. 12 is a diagram illustrating another example of the user interface according to the embodiment of the present disclosure. FIG. 12 illustrates a sixth screen M6 presented to the creator, for example, when "measure the joint opening" is selected as the method of measuring the angle and "selecting from predefined parts" is selected as the selection of the measurement site.

As illustrated in FIG. 12, the content generation device 100 receives designation of a part to be evaluated. In this case, the content generation device 100 receives designation of a joint part in order to measure the opening of the joint.

FIG. 13 is a view illustrating a list example of joint parts according to the embodiment of the present disclosure. The joint part is designated, for example, by being selected by the creator from a pull-down menu displaying a list of joint parts as illustrated in FIG. 13.

As illustrated in FIG. 13, examples of the joint parts that can be designated by the creator include the right elbow and the left shoulder.

When the creator selects a joint part from the pull-down menu in FIG. 13, the selected joint part is highlighted on the human object Ob1 indicating the human body as illustrated in FIG. 12. With this method, the creator can easily perform visual confirmation as to which joint part has been selected.

The selection of the joint part is not limited to the method using the pull-down menu. For example, the creator may select a joint displayed on the human object Ob1 to select the joint part to be the measurement target.

Since the setting of the opening direction of the joint and setting of the threshold are the same as those of the fifth screen M5, the description thereof will be omitted.

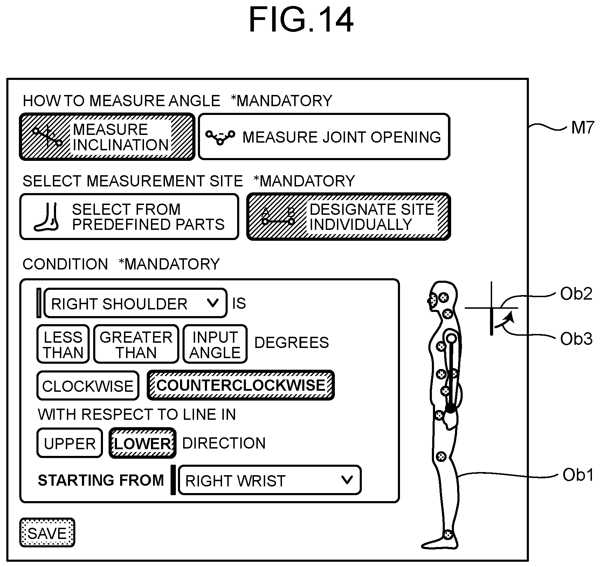

FIG. 14 is a diagram illustrating another example of the user interface according to the embodiment of the present disclosure. FIG. 14 illustrates a seventh screen M7 presented to the creator, for example, when "measure the inclination" is selected as the method of measuring the angle and "individually designate site" is selected as the selection of the measurement site.

As illustrated in FIG. 14, the content generation device 100 receives designation of a site to be evaluated. In this case, the content generation device 100 receives designation of two sites in order to measure the inclination.

The creator selects a site from the pull-down menu in FIG. 11, for example. With this method, as illustrated in FIG. 11, the selected site is highlighted on the human object Ob1. With this method, the creator can easily perform visual confirmation as to which site has been selected.

In addition, on the seventh screen M7, the creator can designate the inclination direction. In the example of FIG. 14, the creator designates whether the line connecting the designated sites is clockwise or counterclockwise with respect to a reference line (here, upward or downward).

At this time, for example, the content generation device 100 displays a line object Ob2 indicating a reference line and an arrow object Ob3 indicating an inclination direction on the seventh screen M7. With this method, the creator can easily perform visual confirmation as to which direction has been selected with respect to which reference line.

Since the setting of the threshold is the same as that of the fifth screen M5, the description thereof will be omitted.

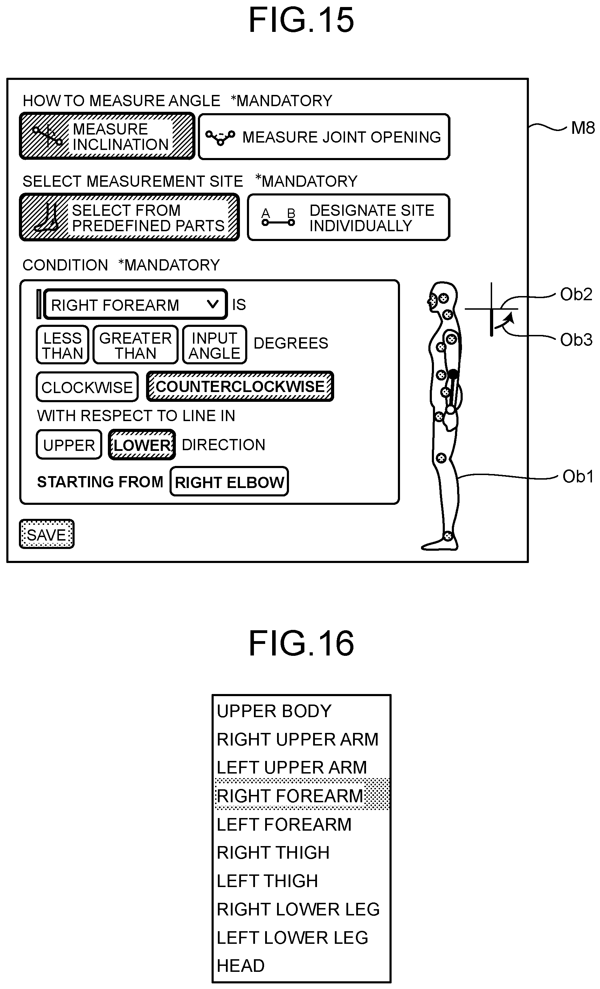

FIG. 15 is a diagram illustrating another example of the user interface according to the embodiment of the present disclosure. FIG. 15 illustrates an eight screen M8 presented to the creator, for example, when "measure the inclination" is selected as the method of measuring the angle and "selecting from predefined parts" is selected as the selection of the measurement site.

As illustrated in FIG. 15, the content generation device 100 receives designation of a part to be evaluated. In this case, the content generation device 100 receives designation of the site part in order to measure the inclination.

FIG. 16 is a view illustrating a list example of site parts according to the embodiment of the present disclosure. The site part is designated, for example, by being selected by the creator from a pull-down menu displaying a list of site parts as illustrated in FIG. 16. Examples of the site parts that can be designated by the creator include the head, upper body, and right upper arm.

For example, the creator selects a site part from the pull-down menu in FIG. 16. With this method, as illustrated in FIG. 15, the selected site is highlighted on the human object Ob1. With this method, the creator can easily perform visual confirmation as to which site part has been selected.

Since the setting of the inclination direction and the threshold is the same as that of the seventh screen M7, the description thereof will be omitted.

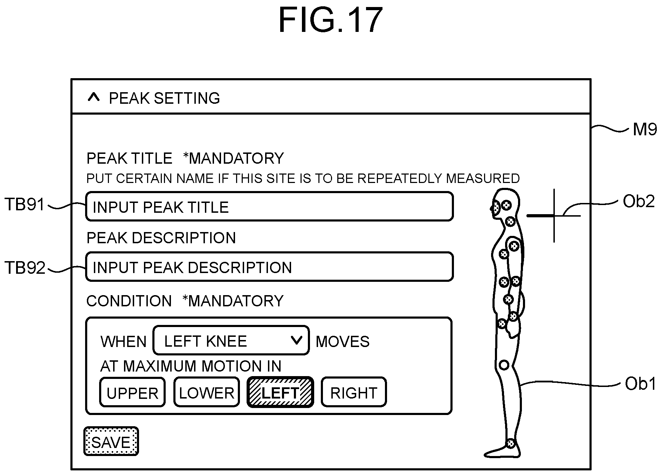

FIG. 17 is a diagram illustrating another example of the user interface according to the embodiment of the present disclosure. FIG. 17 illustrates a ninth screen M9 presented to the creator when the determination item region R22 is selected in a state where, for example, "at maximum motion" is selected as the determination method. The ninth screen M9 may be displayed by transitioning from the second screen M2 (refer to FIG. 7), or the ninth screen M9 may be displayed on a part of the second screen M2 or superimposed on the second screen M2.

On the ninth screen M9, the content generation device 100 receives an input of an evaluation formula of a determination item for determining a peak.

As illustrated in FIG. 17, on the ninth screen M9, the content generation device 100 receives an input of a title (peak title) of a determination item to be newly created, at a text box TB91. In addition, the content generation device 100 receives an input of description of the condition (peak description) in a text box TB92.

The creator can generate a new evaluation formula using the evaluation formula previously created or the evaluation formula created by another person. Although not illustrated here, for example, by inputting a peak title in a text box as in FIG. 9, the creator can use an existing evaluation formula as a template. Alternatively, the creator may select an existing evaluation formula from a pull-down menu.

As illustrated in FIG. 17, the content generation device 100 receives designation of a site to be evaluated as a condition for peak determination. In this case, the content generation device 100 receives the input of the site using a pull-down menu displaying a list of sites as illustrated in FIG. 11, for example.

When the creator selects a site from the pull-down menu in FIG. 11, the selected site is highlighted on the human object Ob1 indicating the human body as illustrated in FIG. 17. With this method, the creator can easily perform visual confirmation as to which site has been selected.

The selection of the site is not limited to the method using the pull-down menu. For example, the creator may select one or a plurality of sites displayed on the human object Ob1 (a position indicated by a circle of the human object Ob1 in FIG. 17) to select the site to be the measurement target.

In addition, as illustrated in FIG. 17, on the ninth screen M9, the creator can designate the direction in which the peak is determined, that is, the direction in which the site moves. In the example of FIG. 17, the creator designates upper, lower, left, and right directions to designate a direction in which the peak is determined.

At this time, for example, the content generation device 100 displays, on the ninth screen M9, a line object Ob2 indicating a direction in which the peak is determined. The content generation device 100 can highlight the direction selected by the creator in the line object Ob2. With this method, the creator can easily perform visual confirmation as to which direction has been selected.

The screen presented to the creator when the determination item region R22 is selected in a state where "at designated angle" is selected as the determination method is the same as the screen described with reference to FIGS. 9 to 16, and thus the description thereof is omitted.

In addition, although the method of inputting one determination item has been described here, it is also allowable to set a plurality of determination items to be input by the creator. In this case, for example, the creator can select a determination method and clicking the determination item region R22 to add a new determination item (refer to FIG. 7).

In addition, although the input method of inputting one evaluation formula in one determination item has been described here, it is also allowable to set a plurality of evaluation formulas to be input by the creator. In this case, the plurality of evaluation formulas can be expressed as one evaluation formula using a logical operator. For example, the evaluation formula #1 and the evaluation formula #2 can be expressed as an evaluation formula such as "evaluation formula #1 AND evaluation formula #2" or " evaluation formula #1 OR evaluation formula #2" using a logical product or a logical sum. The similar applies to three or more evaluation formulas.

In this manner, in the present embodiment, the creator inputs the designation of the measurement site to be analyzed, the measurement method (angle and distance), and the determination condition (threshold). The content generation device 100 generates the evaluation formula of the determination item in accordance with the input of the creator. In this manner, the content generation device 100 sets the motion analysis definition (configuration).

With this method, the creator can more easily create the motion analysis definition without creating the evaluation formula.

Next, a user interface presented to the creator when the content generation device 100 generates the feedback Item will be described.

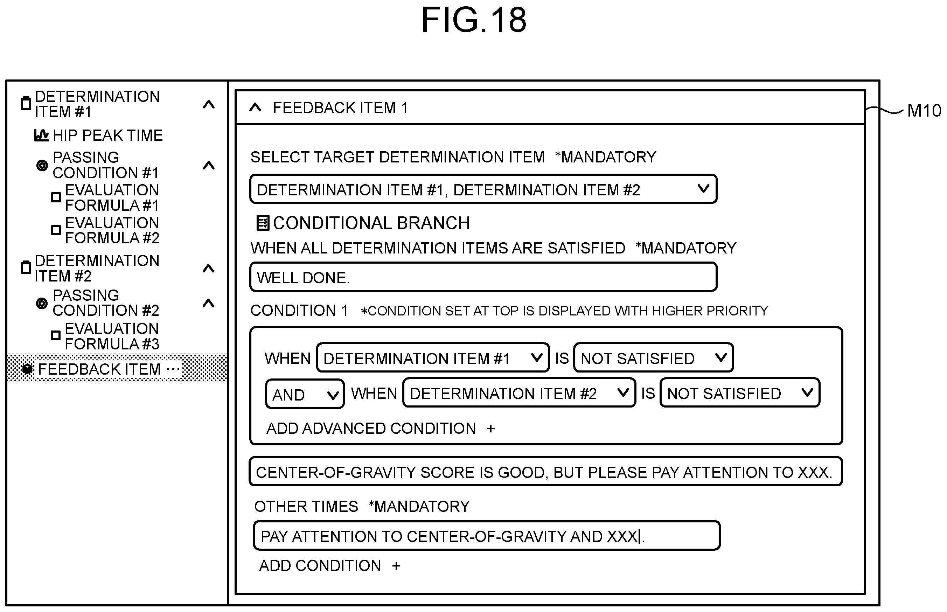

FIG. 18 is a diagram illustrating another example of the user interface according to the embodiment of the present disclosure. FIG. 18 illustrates, for example, a tenth screen M10 presented to the creator when the feedback item region R12 (refer to FIG. 6) is selected (by clicking, for example). The tenth screen M10 may be displayed by transitioning from the first screen M1, or the tenth screen M10 may be displayed on a part of the first screen M1 or superimposed on the first screen M1.

As illustrated in FIG. 18, the creator selects a target determination item and inputs a feedback item. The creator inputs a message in a case where a condition is satisfied or not satisfied in one or more determination items that have been defined.

When the target determination item exists in plurality, the creator can set a conditional branch in accordance with a combination of whether each determination item satisfies a condition, and can set a message in accordance with the combination.

For example, when inputting a feedback item for two determination items #1 and #2, the creator inputs a message to be displayed in a case where determination item #1 is not satisfied and determination item #2 is not satisfied. In addition, the creator inputs a message to be displayed in other cases.

In addition to the above, the creator can input a message in accordance with a combination of determination items, such as a message displayed when determination item #1 is satisfied or a message displayed when determination item #1 is not satisfied and determination item #2 is satisfied.

The content generation device 100 generates the feedback Item based on the input of the creator.

As described above, the content generation device 100 generates the motion analysis definition including the determination item definition and the feedback Item.

### [Video upload processing]

Returning to FIG. 4, having executed the motion analysis definition processing and generated the motion analysis definition, the content generation device 100 next executes video upload processing, and receives the upload of the video from the creator. With this procedure, the content generation device 100 acquires the training video from the creator.

For example, as illustrated in FIG. 6, in the content generation device 100, the creator selects "video list" on the right side on the first screen M1 to present a list of videos held by the content generation device 100 to the creator. For example, the content generation device 100 acquires a video selected from the video list by the creator as a verification video to be described below. On the other hand, in a case where the video to be used for verification is not included in the video list, the content generation device 100 receives upload of the video from the creator.

As illustrated in FIG. 4, the content generation device 100 acquires at least one of a full-score posture video, a satisfactory posture video, and a non-satisfactory posture video for example, as the verification video. The full-score posture video is a training video satisfying all determination items. The non-satisfactory posture video is a training video that does not satisfy at least one of determination items. The satisfactory posture video is a training video that satisfies at least one threshold but does not satisfy the other threshold(s) in a case where a plurality of thresholds is set for one determination item.

For example, it is assumed that, in the determination item of determining the height of the left thigh when the left leg is lifted, the first evaluation formula that the angle is smaller than 10 degrees that is the first threshold with respect to the line in the upward (horizontal) direction is set, and the second evaluation formula that the angle is smaller than 30 degrees that is the second threshold is set.

In this case, the training video satisfying the first evaluation formula is determined as the full-score posture video. In addition, the training video that does not satisfy the first threshold but satisfies the second evaluation formula is determined as the satisfactory posture video. In addition, the training video that does not satisfy any of the first threshold and the second threshold is determined as the non-satisfactory posture video.

Alternatively, in a case where a plurality of determination items is defined, it is also allowable to have a criterion such that a training video satisfying all the determination items is to be determined as a full-score posture video, and a training video satisfying a predetermined number or more and less than all (for example, two or more and less than four determination items when a total number of determination items is five) is to be determined as the satisfactory posture video. In this case, the training video satisfying less than the predetermined number of determination items is to be determined as the non-satisfactory posture video.

Alternatively, when the determination item is defined in plurality, it is also allowable to have a criterion such that the training video satisfying all the determination items is to be the full-score posture video, and the training video satisfying a specific determination item is to be determined as the satisfactory posture video. In this case, the training video that does not satisfy the specific determination item is to be determined as the non-satisfactory posture video. The determination items other than the specific determination items do not contribute to pass/fail (satisfactory/non-satisfactory) determination of the training, but are determination items that are to be deducted from the total score or are to returned to the patient as feedback as future challenges, for example.

In this manner, the content generation device 100 can acquire the verification video (for example, videos such as a full-score posture video, a satisfactory posture video, and a non-satisfactory posture video) in a different situation. This enables the content generation device 100 to verify whether the motion analysis definition is generated as intended by the creator in various situations.

### [Feedback verification processing]

Next, the content generation device 100 executes feedback verification processing. The content generation device 100 executes feedback verification processing using the acquired verification video.

The content generation device 100 presents the verification result to the creator. The creator performs correction and reconfiguration of the motion analysis definition based on the verification result.

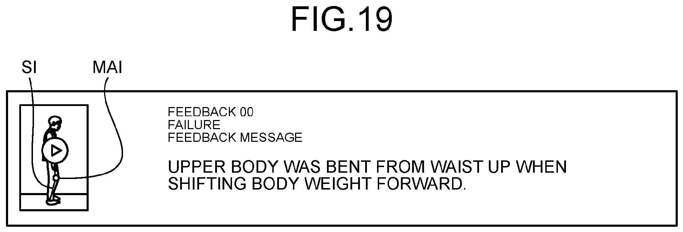

FIG. 19 is a diagram illustrating an example of a verification result according to the embodiment of the present disclosure. When analyzing the verification video using the motion analysis definition, the content generation device 100 presents a first verification screen including a feedback message obtained as an output, to the creator. The first verification screen is, for example, the same screen as the screen used as feedback to the patient when the patient actually images the training state and performs motion analysis.

The feedback result included in the first verification screen is information indicating whether the training is a success or failure. The example of FIG. 19 illustrates a case where the feedback result was a "failure".

In this case, the content generation device 100 displays a message corresponding to the failure details, that is, the determination result of the determination item. This message is predefined as a feedback Item. The example of FIG. 19 displays a message "Upper body bent from waist up when shifting body weight forward". Alternatively, the content generation device 100 may convert the message into spoken audio.

By confirming the first verification screen, the creator can confirm whether a message matching the content of the verification video is returned as feedback. For example, when the message does not match the details of the verification video, the creator can change the message (feedback Item).

Furthermore, as illustrated in FIG. 19, the first verification screen includes a video (verification video in this case) capturing the training performed by the evaluation target person. At this time, the content generation device 100 displays skeleton information SI (an example of posture information) of the evaluation target person (for example, a person performing training in the verification video) in superimposed display on the evaluation target person. For example, the content generation device 100 selectively displays the skeleton information SI corresponding to the site of the evaluation target person to be subjected to the motion analysis.

In addition, the content generation device 100 displays analysis site information MAI (an example of analysis information) indicating a site of the evaluation target person to be subjected to the motion analysis in superimposed display on the evaluation target person.

The first verification screen illustrated in FIG. 19 is an example. The creator can change which information is to be returned as feedback to the evaluation target person based on the first verification screen. For example, the creator can select and change the skeleton information SI and the analysis site information MAI to be superimposed on the video. In this manner, the creator can predefine a presentation method in a case where the result of the motion analysis (evaluation) is presented to the evaluation target person. For example, the content generation device 100 can generate the individual content using predefining information predefining the presentation method as the feedback Item.

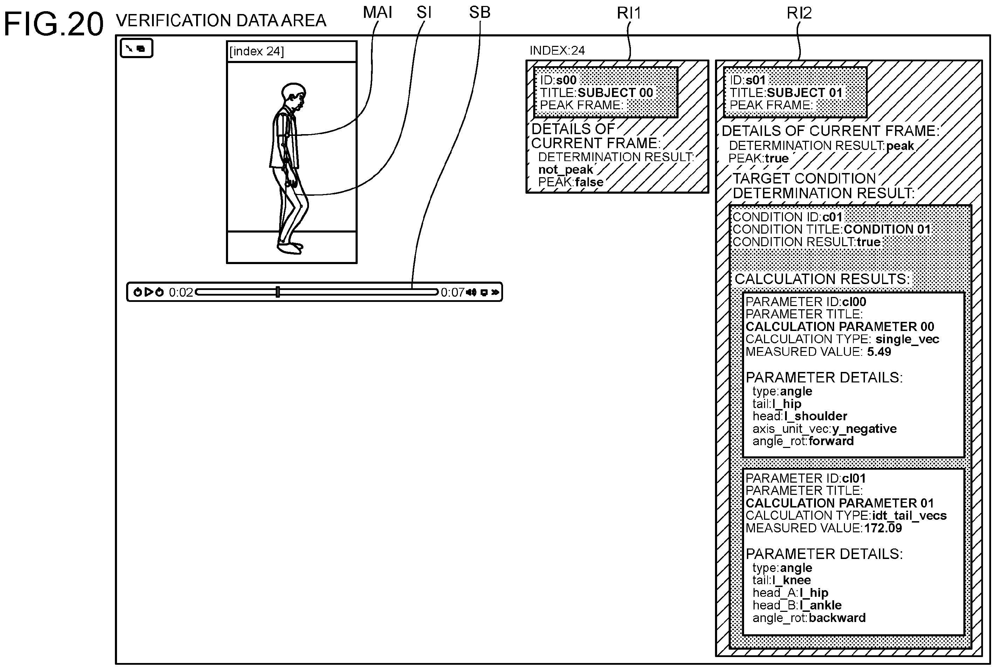

FIG. 20 is a diagram illustrating another example of the verification result according to the embodiment of the present disclosure. The content generation device 100 presents, to the creator, a second verification screen including details of each determination item in a case where the verification video is analyzed using the motion analysis definition.

Furthermore, as illustrated in FIG. 20, the second verification screen includes a video (verification video in this case) capturing the training performed by the evaluation target person. At this time, the content generation device 100 displays skeleton information SI of the evaluation target person (for example, a person performing training in the verification video) in superimposed display on the evaluation target person. For example, the content generation device 100 selectively displays the skeleton information SI corresponding to the site of the evaluation target person to be subjected to the motion analysis.

In addition, the content generation device 100 displays analysis site information MAI indicating a site of the evaluation target person to be subjected to the motion analysis in superimposed display on the evaluation target person.

The content generation device 100 displays a seek bar SB together with the video. The creator can operate the seek bar SB to change the play position of the video.

Furthermore, the content generation device 100 displays result information RI indicating an analysis result of the motion, for example, in a state where the video is stopped. The content generation device 100 displays the analysis result of the motion in a frame at a timing when the video is stopped, as the result information RI. INDEX included in the second verification screen indicates a frame number of a video, for example.

The result information RI is displayed for each determination item, for example. In the example of FIG. 20, two pieces of result information RI1 and RI2 are illustrated.

The result information RI1 includes information related to a determination item title and preconditions. The information related to the precondition includes, for example, information related to a timing at which determination is performed. Here, examples of the timing of the determination include "make determination at any time", "at maximum motion", and "at a designated angle". The example of FIG. 20 illustrates that the determination is to be made at the "at maximum motion", that is, at the peak time.

The result information RI1 includes information indicating a determination result of the precondition, that is, whether a peak has been detected or whether it has been determined that it is the timing to determine the determination item (evaluation formula). The determination result of the precondition is indicated as a determination result of "Details of current frame", for example. In the example of FIG. 20, the timing determination result indicates "not_peak", with the peak detection result "peak" indicating "false". This indicates that the content generation device 100 has not detected a peak and has determined that the current frame does not correspond to the peak of motion.

The result information RI2 includes the determination result of the precondition similarly to the result information RI1. In the example of FIG. 20, the timing determination result indicates "peak", with the peak detection result "peak" indicating "true". This indicates that the content generation device 100 has detected a peak and has determined that the current frame corresponds to the peak of motion.

In this case, the content generation device 100 determines the condition (evaluation formula). The content generation device 100 presents the determination result of the evaluation formula to the creator as a target condition determination result. The target condition determination result includes a condition title and a condition result being a determination result of the passing condition. The example of FIG. 20 illustrates a case where the condition result is "true" and the passing condition is satisfied, that is, the determination item is satisfied.

In addition, the target condition determination result includes a calculation result of the evaluation formula. For example, the content generation device 100 presents information related to parameter included in the evaluation formula to the creator as the calculation result.

For example, when the content generation device 100 determines whether the angle of the joint defined by the designation of the body site exceeds a threshold, the content generation device 100 makes the determination using an evaluation formula with the angle of the joint set as a parameter.

In this case, the content generation device 100 displays the parameter title, the calculation type (information indicating whether to measure inclination or whether to measure opening of joint), the value of the actually calculated joint angle (measured value), and the like, as the information related to parameter.

The content generation device 100 can also present details of the parameters to the creator. For example, the content generation device 100 displays a parameter type (an angle, a distance, or the like), information predefining the parameter, and the like, as details of the parameter.

The information predefining the parameter includes information related to a start point and an end point when the type of the parameter is a distance, for example. For example, when the distance between the right hand and the right shoulder is a parameter, the information predefining the parameter includes, for example, information indicating that the start point (head) of the parameter is the right shoulder and the end point (tail) is the right hand.

Furthermore, for example, in the case of an angle representing the inclination of the site, the information predefining the parameter includes information indicating the site (for example, a start point and an end point), information to be a reference of the angle (for example, a coordinate axis), and information related to a direction of the angle (for example, a rotation direction).

When the angle formed by the line connecting the left hip and the left shoulder and the axis in the y negative direction is set as a parameter, the information predefining the parameter includes information indicating that the start point (head) of the straight line to measure the inclination is the left shoulder (1-shoulder) and the end point (tail) is the left hip (1_hip). The information predefining the parameter includes information indicating that the reference axis is in the y-axis negative direction (y_negative). The information predefining the parameter includes information indicating that the angle direction (angle_rot) is a forward direction (forward). The information indicating the direction of the angle may be information indicating clockwise or counterclockwise instead of the forward direction.

Furthermore, in the case of an angle indicating opening of the joint, the information predefining the parameter includes, for example, information indicating a site (for example, a start point and an end point) and information related to a direction of the angle (for example, a rotation direction).

In a case where the angle between the line connecting the left knee and the left hip and the line connecting the left knee and the left ankle is set as a parameter, the information predefining the parameter includes information indicating that the start point (head_A, head B) of the two lines is the left hip (l_hip) and the left ankle (l_ankle) and the end point (tail) is the left knee (l_knee). The information predefining the parameter includes information indicating that the angle direction (angle_rot) is a forward direction (forward). The information indicating the direction of the angle may be information indicating clockwise or counterclockwise instead of the forward direction.

Although the case where the parameter is designated by the individual site has been described here, the parameter may be designated by a predefined site. In this case, the information predefining the parameter may include information predefining the part (for example, left knee (l_knee)).

Furthermore, the content generation device 100 may present information other than the above-described result information RI to the creator. For example, the content generation device 100 can present an evaluation formula, an evaluation result (true or false) of the evaluation formula, and the like to the creator.

In this manner, for example, the content generation device 100 performs analysis using the motion analysis definition for each frame of the verification video, and displays the analysis result for each frame in accordance with the instruction of the creator. With this method, the creator can confirm the analysis result obtained by the content generation device 100 for each frame, and can confirm whether the motion analysis definition is correctly set.

At this time, the content generation device 100 may display the measured value in the current frame as the information related to parameter, and may receive editing of the threshold in the display. With this method, the creator can correct (edit) the threshold of the evaluation formula while confirming the measured value. For example, in a case where the result of the threshold determination of the evaluation formula is true in the current frame, the creator can change the threshold such that the result of the threshold determination is false.

Furthermore, for example, the content generation device 100 may display information indicating a timing at which the precondition or the passing condition is satisfied or information indicating the determination disregarded section to be superimposed on the seek bar SB.

By confirming the second verification screen, the creator can confirm whether the motion analysis definition is correctly set. For example, when the motion analysis definition is not set correctly, the creator can change the motion analysis definition.

Here, the content generation device 100 analyzes the verification video using the motion analysis definition, but the device that performs the analysis is not limited to the content generation device 100. For example, an external analysis device (not illustrated) may acquire the motion analysis definition and the verification video from the content generation device 100 and perform the analysis. The content generation device 100 acquires an analysis result from the analysis device and presents the analysis result to the creator. Note that the analysis device can also analyze actual training by the patient.

### [Content generation/output processing]

When the feedback verification processing ends, the content generation device 100 generates individual content and outputs the generated individual content to the content providing device 200. The individual content includes, for example, information related to a training description, a training video (accompany video) and/or a training audio (accompany audio), and information related to the motion analysis definition (checking perspective definition).

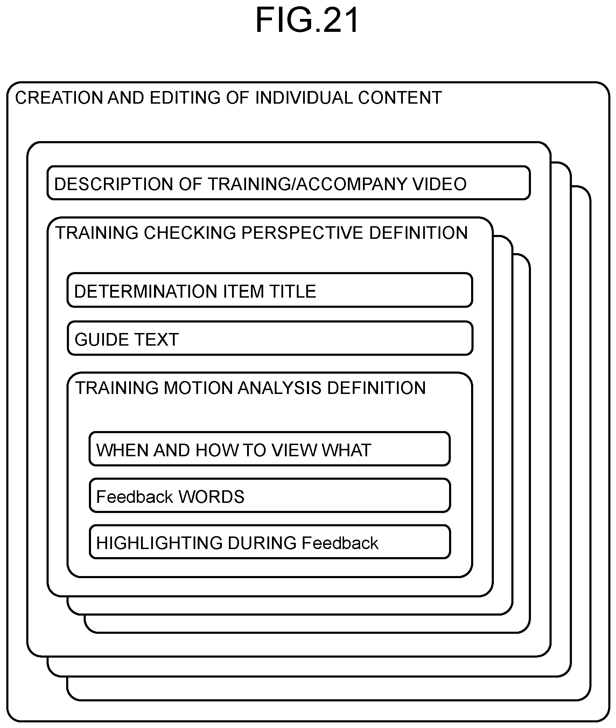

FIG. 21 is a diagram illustrating an example of individual content according to the embodiment of the present disclosure. As illustrated in FIG. 21, the content generation device 100 generates individual content including information related to training description and a training video (accompany video).

Furthermore, the content generation device 100 generates individual content including the training checking perspective definition. The checking perspective definition includes a determination item title, a guide text indicating the determination item details, and information related to a training motion analysis definition. The checking perspective definition may be set in plurality for one piece of individual content. The checking perspective definition can be set for each determination item, for example.

The motion analysis definition includes information related to when, how, and what to view, that is, preconditions and passing conditions. Furthermore, the motion analysis definition includes feedback words, that is, information related to the feedback Item.

Furthermore, the motion analysis definition may include information related to how to apply highlighting during feedback. This is information predefining a feedback method when the analysis result is returned as feedback to the evaluation target person. For example, the information related to the highlighting method includes information indicating whether to display the skeleton information SI and the analysis site information MAI when performing feedback of the analyzed video.

The content generation device 100 outputs the generated individual content to the content providing device 200.

### <2.3. Another example of content generation device>

In the content generation device 100 described above, the threshold is set based on the input by the creator, but the threshold may be set by the content generation device 100. Hereinafter, a content generation device 100A that performs setting of the threshold will be described.

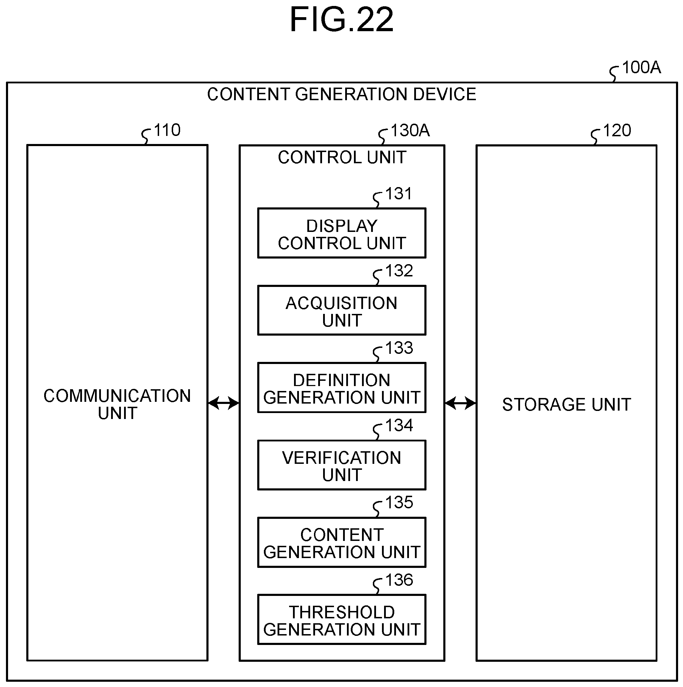

FIG. 22 is a block diagram illustrating a composition example of the content generation device 100A according to the embodiment of the present disclosure. The content generation device 100A illustrated in FIG. 22 has a composition similar to the composition of the content generation device 100 illustrated in FIG. 3 except that a control unit 130A includes a threshold generation unit 136.

The threshold generation unit 136 generates a threshold of the passing condition. For example, the threshold generation unit 136 acquires a full-score posture video that satisfies the passing condition and a non-satisfactory posture video that does not satisfy the passing condition via the acquisition unit 132. The threshold generation unit 136 generates a threshold using the acquired satisfactory posture video and non-satisfactory posture video.

For example, the threshold generation unit 136 generates a threshold using the threshold determination model. For example, the threshold determination model uses a data set including a satisfactory video and a non-satisfactory video as an input, and outputs the threshold. The threshold determination model is generated using machine learning such as deep neural network (DNN). The threshold determination model is generated based on supervised image data including a full-score posture video and a non-satisfactory posture video, for example.

Alternatively, the threshold generation unit 136 may generate the threshold using the skeleton estimation model and the threshold calculation model. The skeleton estimation model uses the satisfactory posture video or the non-satisfactory posture video as an input, and outputs skeleton information of the evaluation target person. In addition, the threshold calculation model uses skeleton information output by the skeleton estimation model as an input, and outputs a threshold related to a precondition of a determination item. The threshold calculation model calculates the threshold of the passing condition based on the skeleton information regarding the satisfactory video and the skeleton information regarding the non-satisfactory video.

Here, it is assumed that the threshold generation unit 136 generates one threshold, but the threshold generation unit 136 may generate a plurality of thresholds. In this case, for example, the threshold generation unit 136 generates a plurality of thresholds using the satisfactory posture video in addition to the full-score posture video and the non-satisfactory posture video.

For example, the threshold generation unit 136 determines that the passing condition is satisfied in the posture of the full-score posture video, and calculates the first threshold for determining that the passing condition is not satisfied in the posture of the satisfactory posture video. Furthermore, the threshold generation unit 136 determines that the passing condition is satisfied in the posture of the satisfactory posture video, and calculates a second threshold for determining that the passing condition is not satisfied in the posture of the non-satisfactory posture video.

The threshold generation unit 136 outputs the generated threshold to the definition generation unit 133. The definition generation unit 133 sets the threshold generated by the threshold generation unit 136 to generate an evaluation formula.

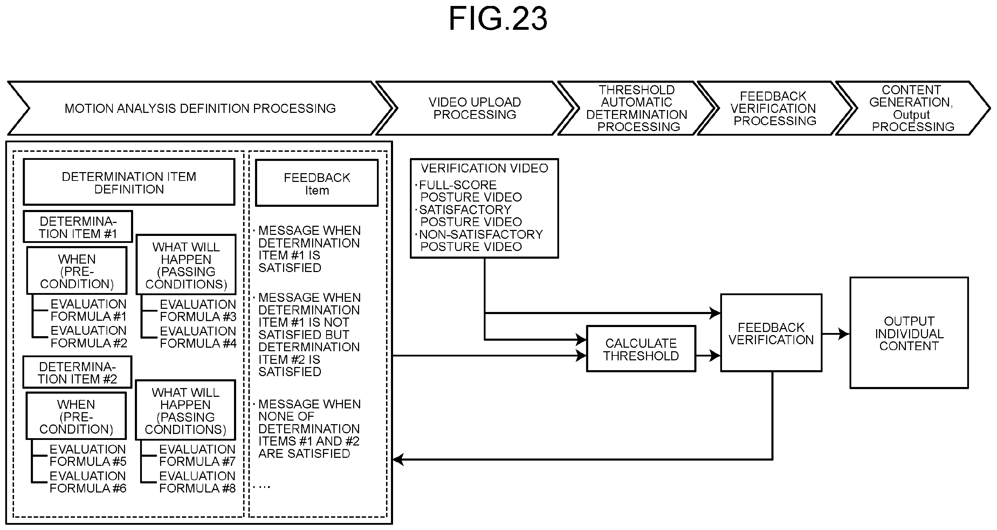

FIG. 23 is a diagram illustrating another example of individual content generation processing according to the embodiment of the present disclosure. The individual content generation processing illustrated in FIG. 23 includes the same processing as the individual content generation processing illustrated in FIG. 23 except that the processing includes threshold automatic determination processing.

In motion analysis definition processing, the content generation device 100 that has defined the determination items excluding the threshold acquires at least one of the full-score posture video, the satisfactory posture video, and the non-satisfactory posture video in the video upload processing.

Next, the content generation device 100 executes threshold automatic determination processing, and calculates a threshold using at least one of the full-score posture video, the satisfactory posture video, and the non-satisfactory posture video. The content generation device 100 sets the motion analysis definition using the calculated threshold, and executes feedback verification processing using the set motion analysis definition.

In this manner, by setting the threshold automatically by the content generation device 100, the creator can set the motion analysis definition without setting the threshold. With this method, the creator can generate the individual content more easily.

### <<3. Content providing device>>

Next, the content providing device 200 will be described. The content providing device 200 is an information processing device that generates prescription content (an example of edited content) using individual content and provides generated the prescription content to a patient. The content providing device 200 may be an edge computer that performs edge processing near the user, or may be a cloud computer that performs processing on the cloud side, that is, may be a server device.

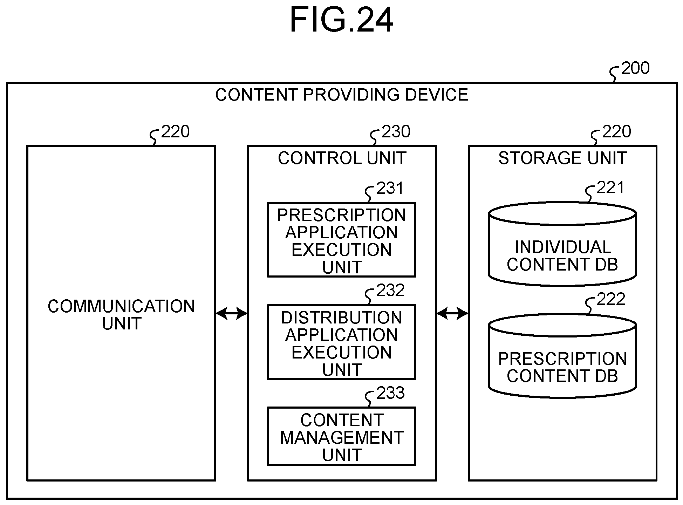

FIG. 24 is a block diagram illustrating a composition example of the content providing device 200 according to the embodiment of the present disclosure. The content providing device 200 includes a communication unit 210, a storage unit 220, and a control unit 230. Note that the composition illustrated in FIG. 24 is a functional composition, and the hardware composition may be different from this. Furthermore, the functions of the content providing device 200 may be implemented in a distributed manner in a plurality of physically separated structures. For example, the content providing device 200 may be constituted with a plurality of server devices.

### [Communication unit 210]

The communication unit 210 is a communication interface for communicating with other devices. The communication unit 210 may be a network interface or a device connection interface. For example, the communication unit 210 may be a local area network (LAN) interface such as a network interface card (NIC), or may be a universal serial bus (USB) interface including a USB host controller, a USB port, and the like. Furthermore, the communication unit 210 may be a wired interface, or may be a wireless interface.

The communication unit 210 functions as a communication means used in the content providing device 200. The communication unit 210 communicates with the content generation device 100, the information processing device 400 used by a medical person on the hospital side, and the information processing device 500 used by the patient.

### [Storage unit 220]

The storage unit 220 is a data readable/writable storage device such as dynamic random access memory (DRAM), static random access memory (SRAM), a flash drive, or a hard disk. The storage unit 220 functions as a storage means in the content generation device 100.

The storage unit 220 includes an individual content DB 221 and a prescription content DB 222. The individual content DB 221 stores the individual content generated by the content generation device 100. The prescription content DB 222 stores the prescription content generated by the content providing device 200.

### [Control unit 230]

The control unit 230 is implemented by execution of various programs stored in a storage device inside the content providing device 200 by a CPU, an MPU, or the like, using RAM as a work area. Furthermore, the control unit 230 is implemented by an integrated circuit such as ASIC or FPGA.

As illustrated in FIG. 24, the control unit 230 includes a prescription application execution unit 231, a distribution application execution unit, and a content management unit 233, and implements or executes functions and actions of information processing described below. Not limited to the composition illustrated in FIG. 24, the internal composition of the control unit 230 may have a different composition as long as it performs information processing described below. Furthermore, the connection relationship of the processing units included in the control unit 230 is not limited to the connection relationship illustrated in FIG. 24, and may be a different connection relationship.

### (Prescription application execution unit 231)

The prescription application execution unit 231 generates prescription content in accordance with an instruction from a medical person on the hospital side. The prescription content includes at least one piece of individual content.

For example, the medical person executes a prescription application preinstalled in the information processing device 400 used by the medical person, and inputs information related to prescription content via the prescription application. The prescription application execution unit 231 acquires information related to prescription content from a medical person via the prescription application executed on the information processing device 400. The prescription application execution unit 231 generates prescription content based on the acquired information related to the prescription content.

The prescription application can be an application that operates on an arbitrarily chosen operation system (OS) installed in the information processing device 400 on the hospital side.

Alternatively, the medical person may input information related to the prescription content using an arbitrarily chosen browser as an interface. The prescription application execution unit 231 generates prescription content based on information related to the prescription content acquired via the browser.

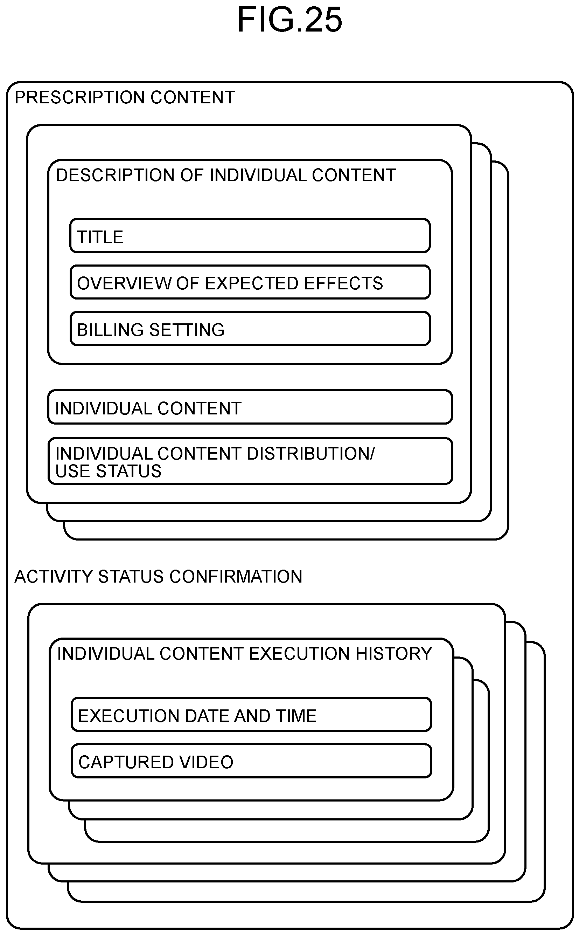

FIG. 25 is a diagram illustrating an example of a user interface of a prescription application according to the embodiment of the present disclosure. FIG. 25 illustrates a prescription application screen to be presented to the medical person on the hospital side when the prescription application is executed by the prescription application execution unit 231, for example.

The prescription application screen illustrated in FIG. 25 displays information related to prescription content. The prescription content includes one or more pieces of individual content. FIG. 25 illustrates a case where the prescription content three pieces of individual content.

Examples of the information related to the prescription content displayed on the prescription application screen include information related to individual content included in the prescription content. The information related to the individual content displayed on the prescription application screen includes, for example, information related to the individual content, information related to the motion analysis definition, and information related to the distribution/use status of the individual content.

The information related to the individual content includes, for example, a title of the individual content and an outline of an effect expected by executing training included in the individual content. For example, in a case where a fee is required for use of the individual content, information related to the fee (billing setting) is included in the information related to the individual content.

As described above, the individual content can be created by a creator other than a medical person. In this case, the creator can authorize a third party other than the creator to use the individual content on condition of payment of a fee or the like. Alternatively, the creator can grant use of the individual content with a limited use range, for example, within a hospital. In this manner, the individual content may be made available to a third party other than the creator by satisfying a predetermined condition such as payment of the fee or participation in a group.

The use range can be predefined, for example, for each motion of the evaluation target or for each training content such as rehabilitation or exercise. Alternatively, the use range may be predefined for each evaluation target such as race, gender, and age. Furthermore, the use range may be predefined for each target field of training, such as a medical field or an item of sports, or may be predefined for each country or locational region.

The prescription application screen displays prescription content of a specific patient, for example. Alternatively, the prescription application screen may display prescription content of each of a plurality of patients.

The medical person selects one or more pieces of individual content stored in the individual content DB 221 to generate new prescription content. In addition, the medical person adds new individual content to the existing prescription content or deletes the individual content included in the prescription content to update the prescription content.

In addition, the prescription application execution unit 231 receives editing of individual content included in prescription content from a medical person. For example, the medical person edits the individual content in accordance with the condition of the patient. Specifically, the prescription application execution unit 231 receives a change in a threshold, a training time, or the like included in the motion analysis definition of the individual content, from the medical person.

The prescription application screen further displays information confirming the activity status of the patient. The information confirming the activity status of the patient includes, for example, information related to an execution history of the individual content. The information related to the execution history of the individual content is generated for each individual content included in the prescription content.

The information related to the execution history of the individual content includes, for example, information related to an execution date and time of the individual content and a captured video obtained by imaging a state of the training.

The prescription application screen displays information confirming the activity status of a specific patient, for example. Alternatively, the prescription application screen may display information confirming the activity status of each of the plurality of patients.

The medical person generates prescription content based on an examination of a visiting patient, for example. In addition, at the next visit, the medical person generates new prescription content while referring to the prescription content execution history of the patient. In addition, the medical person, together with the patient, can confirm the captured video obtained by imaging the state of the training. This allows the medical person to perform better consultation on the patient.

Note that the medical person may examine the patient through online medical examination or the like and generate the prescription content. In addition, the medical person may generate the prescription content in the presence of the patient such as at the time of examination of the patient, or may generate the prescription content in the absence of the patient.

Furthermore, here, the medical person selects one or more pieces of individual content stored in the individual content DB 221, but the method of selecting the individual content is not limited thereto. For example, a medical person may cause a prescription application to scan various codes such as a QR code (registered trademark) to select individual content and generate prescription content. Here, the various codes may be, for example, a code including identification information designating individual content or a code including identification information designating prescription content including at least one piece of individual content. Alternatively, the medical person may input information identifying the patient to the prescription application to select individual content associated with the patient. Examples of the information identifying the patient include a name of the patient, an insurer number, and a patient ID allocated for each hospital, for example.

### (Distribution application execution unit 232)

The distribution application execution unit 232 distributes the prescription content in accordance with an instruction from the patient. The distribution application execution unit 232 acquires information designating prescription content from the patient. The information designating the prescription content may be information corresponding to the prescription content (for example, various codes to be described below) or may be information related to the patient (for example, information identifying the patient to be described below). The distribution application execution unit 232 transmits prescription content corresponding to the acquired information to the patient.

For example, the patient executes a distribution application preinstalled in the information processing device 400 used by the patient, and acquires prescription content via the distribution app. For example, the patient causes the distribution application to scan various codes such as a QR code (registered trademark) acquired from a medical person, thereby acquiring prescription content prescribed to the patient themselves. Based on various codes acquired from the patient, the distribution application execution unit 232 acquires the prescription content generated for the patient from the prescription content DB 222, and presents the prescription content to the patient.

Alternatively, the patient can input information regarding themselves (for example, information identifying the patient) into the distribution application to acquire the prescription content prescribed for themselves. Examples of the information related to the patient include a name, an insurer number of the patient, and a patient ID allocated for each hospital, for example.

The prescription application may be an application that operates on an arbitrarily chosen operation system (OS) installed in the information processing device 500 used by the patient.

Alternatively, the patient may acquire the prescription content using an arbitrarily chosen browser as an interface. The distribution application execution unit 232 distributes (transmits) the prescription content based on information (for example, various codes) designating the prescription content acquired via the browser.

The prescription content may be stored in the information processing device 500 used by the patient so as to be distributed to the patient, for example, or may be distributed by the patient accessing the prescription content in a state of being stored in the cloud server or the prescription content DB 222.

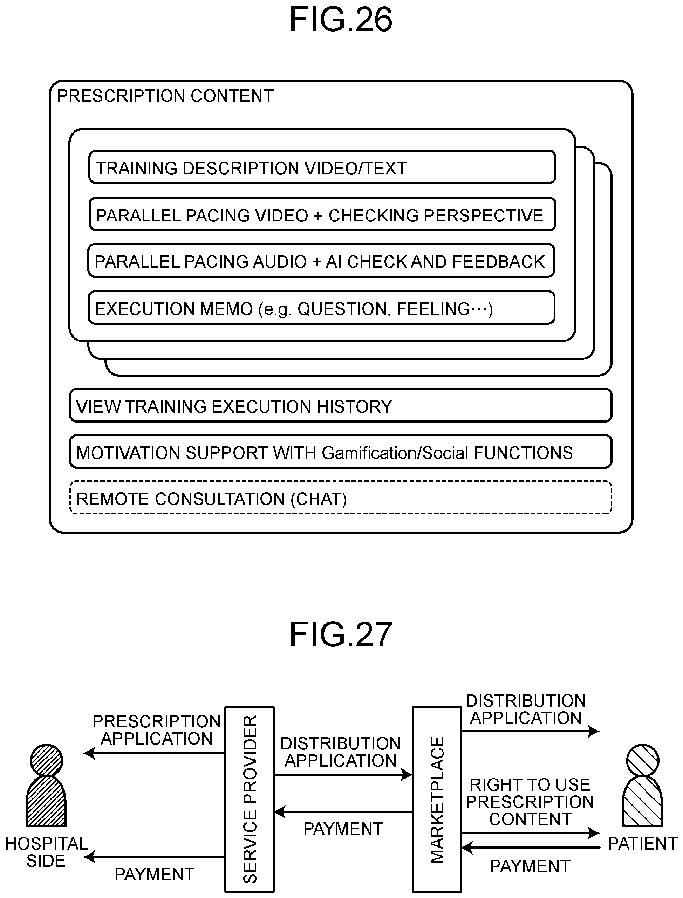

FIG. 26 is a diagram illustrating an example of a user interface of a distribution application according to the embodiment of the present disclosure. FIG. 25 illustrates a distribution application screen to be presented to the patient in a case where the distribution application is executed by the distribution application execution unit 232, for example.

The distribution application screen illustrated in FIG. 26 displays individual distribution information related to the individual content, for each individual content included in the prescription content. When a plurality of pieces of individual content is included in the prescription content, the individual distribution information is displayed for each piece of individual content, for example.

The individual distribution information includes a training reference guide video regarding the prescribed individual content and text information describing the training. In addition, the individual distribution information includes information related to the accompany video to be played when the patient performs the training and information related to a checking perspective of the accompany video. The individual distribution information includes information related to accompany audio to be played when the patient performs training, AI check being an evaluation result of the training result, and feedback. The individual distribution information also includes execution memo information describing feeling, thoughts, and questions held by the patient at the time of training execution.

For example, by selecting the information related to the accompany video and the checking perspectives of the accompany video, the patient can perform the training while confirming the accompany video and the checking perspectives of the accompany video.

For example, by selecting information related to the accompany audio, AI check, and feedback, the patient can perform training in accordance with the accompany audio. Furthermore, by capturing the state of the training performed in accordance with the accompany audio using a camera or the like mounted on the information processing device 500, the patient can check the analysis result of the captured video as AI check and feedback. At this time, the medical person can also confirm the captured video together with the analysis result.

Furthermore, the distribution application screen displays information related to the training execution history and information supporting patient motivation. For example, in FIG. 26, the information related to the training execution history is displayed as a training execution history list. The patient can confirm the training execution history list to confirm which training was performed when.

In addition, as illustrated in FIG. 26, the distribution application can support patient motivation by gamification and social functions. For example, as the gamification function, the distribution application gives a reward or grants a higher level every time patient training is performed. Furthermore, for example, the distribution application allows the patient to share the training execution status and the like with other patients as a social function. With these methods, the distribution application performs support the patient to be able to maintain motivation for the training execution.

Furthermore, the distribution application may be equipped with a function for a patient to make contact with a medical person or the like. In FIG. 26, the distribution application screen includes an icon enabling the patient to perform chat consultation with a medical person. By selecting the icon, the patient can remotely contact the medical person.

### (Content management unit 233)

The content management unit 233 manages individual content and prescription content. The content management unit 233 stores the individual content acquired from the content generation device 100 in the individual content DB 221. The content management unit 233 stores the prescription content generated by the prescription application execution unit 231 in the prescription content DB 222.

In addition, the content management unit 233 manages the use ranges of the individual content and the prescription content (hereinafter, also simply described as content). The content management unit 233 manages whether the content is available to anyone or only some authorized users (medical person or patients).

The use range can be managed, for example, for each motion of the evaluation target or for each training content such as rehabilitation or exercise. Alternatively, the use range may be managed for each evaluation target such as race, gender, and age. Furthermore, the use range may be managed for each target field of training, such as a medical field or an item of sports, or may be predefined for each country or locational region.

In addition, the content management unit 233 manages whether the use of the content is fee-charging or free-of-charge. In a case where the use of the content is fee-charging, the content management unit 233 manages the use fee and the sales of the content. Furthermore, the content management unit 233 may manage the use fee for each user.

Furthermore, the content management unit 233 can manage whether content can be modified (edited or updated) by a person other than the user who created the content.

The content management unit 233 outputs content in accordance with requests from the prescription application execution unit 231 and the distribution application execution unit 232.

As described above, the content generation device 100 of the information processing system 10 according to the present embodiment acquires a checking perspective at the time of evaluating (analyzing) the motion of the patient performing the training. Furthermore, the content generation device 100 determines a motion analysis definition for evaluating patient motion based on the checking perspective. The content generation device 100 acquires the accompany video or accompany audio, and generates individual content including the accompany video or accompany audio and the motion analysis definition.

The content providing device 200 generates prescription content including individual content and distributes (provides) the prescription content to the patient.

With this method, the information processing system 10 can more easily generate the motion analysis definition. Furthermore, the information processing system 10 can more easily generate the individual content including the motion analysis definition.

### <<4. Application example>>

As described above, the prescription application that creates the prescription content and the distribution application that distributes the prescription content are installed and executed in the information processing devices 400 and 500 installed on the hospital side or held by the patient. Here, an example of a method of installing the prescription application and the distribution application will be described.

Hereinafter, it is assumed that a service provider manages and runs the information processing system 10. The following will describe an exemplary case where a hospital sells prescription content to a patient.

FIG. 27 is a diagram illustrating an example of an application installation method according to the embodiment of the present disclosure.

The service provider provides the prescription application to the medical person on the hospital side. The provision of the prescription application may be fee-charging or free-of-charge. In addition, the service provider sells a distribution application on a marketplace, for example.

The patient acquires the distribution application via the marketplace, and purchases the right to use the prescription content by paying a fee. The fee for the right to use the prescription content may be paid for each piece of prescription content or may be paid once when the distribution application is installed. Alternatively, the fee for the right to use the prescription content may be paid every fixed period such as every month.

The marketplace pays the service provider the fee for the right to use the prescription content. At this time, the marketplace may pay the service provider an amount of money remaining after subtracting the fee.

The service provider pays the hospital side for the right to use the prescription content. At this time, the service provider can pay the hospital side for the amount remaining after subtracting the fee.

In this manner, the service provider provides the distribution application to the patient via the marketplace. With this method, the patient can easily acquire the distribution application.

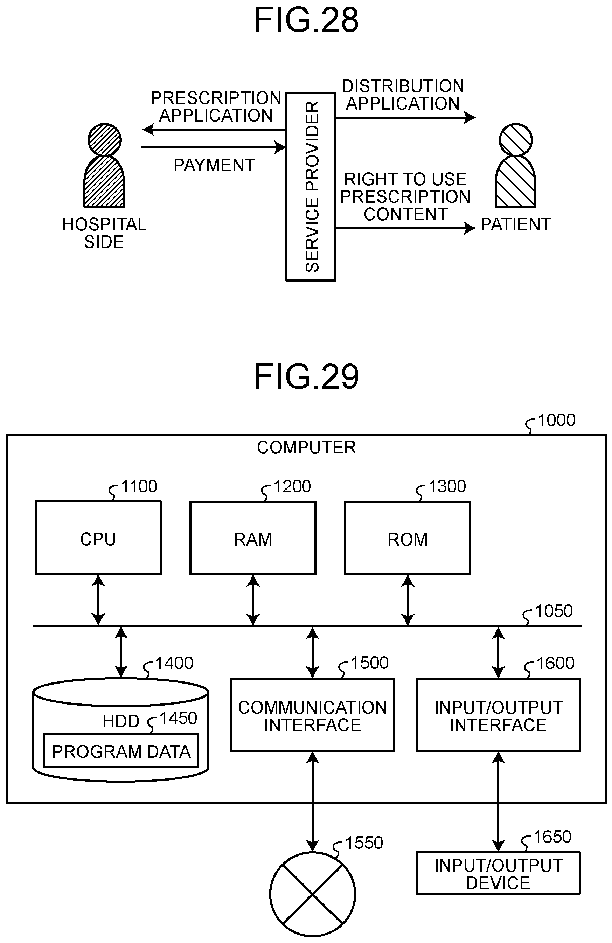

FIG. 28 is a diagram illustrating another example of the application installation method according to the embodiment of the present disclosure.

The service provider provides the prescription application to the medical person on the hospital side. The hospital side pays the service provider for the use fee of the prescription application. The fee may be paid once when the prescription application is installed, or may be paid every fixed period such as every month. Alternatively, the payment may be performed by a usagebased billing method in which the payment is charged in accordance with the usage of the prescription application.

The patient acquires the distribution application and the right to use the prescription content from the service provider. Here, it is assumed that there is no payment from the patient to the service provider. In this case, the patient shall pay for the right to use the prescription content directly to the hospital, for example.

In this manner, the patient can acquire the distribution application from the service provider.

The prescription content can be developed by the hospital side (business entity that receives service provision). At this time, the business entity can restrict the use range of the developed prescription content to its own business entity. That is, a business entity can develop prescription content as closed content with a restricted use range.

With this method, the business entity can differentiate the business by the training skills included in the prescription content.

On the other hand, for example, the business entity can disclose the developed prescription content to other business entities as fee-charging content without restricting the use range of the developed prescription content. That is, a business entity can develop prescription content as shared content with no use range restriction.

With this method, the business entity can expand the revenue channel using the training skills included in the prescription content.

Alternatively, a service provider may develop prescription content. For example, a service provider can develop training content that is not limited to medical purposes, as prescription content. In addition, the service provider can develop, for example, training content for medical care and healthcare, as prescription content.

In this manner, a service provider can differentiate services by developing general-purpose training for many business entities and patients.

Although the development of prescription content has been described as an example here, individual content, motion analysis definition, and the like can be similarly developed by a business entity and/or a service provider.

### <<5. Hardware composition>>

The content generation device 100 and the content providing device 200 according to the present disclosure described above are implemented by a computer 1000 having a composition as illustrated in FIG. 29, for example. FIG. 29 is a hardware composition diagram illustrating an example of the computer 1000 that implements functions of the content generation device 100 according to the present disclosure. The computer 1000 includes a CPU 1100, RAM 1200, read only memory (ROM) 1300, a hard disk drive (HDD) 1400, a communication interface 1500, and an input/output interface 1600. Individual components of the computer 1000 are interconnected by a bus 1050.

The CPU 1100 operates based on a program stored in the ROM 1300 or the HDD 1400 so as to control each of components. For example, the CPU 1100 develops the program stored in the ROM 1300 or the HDD 1400 into the RAM 1200 and executes processing corresponding to various programs.

The ROM 1300 stores a boot program such as a basic input output system (BIOS) executed by the CPU 1100 when the computer 1000 starts up, a program dependent on hardware of the computer 1000, or the like.

The HDD 1400 is a non-transitory computer-readable recording medium that records a program executed by the CPU 1100, data used by the program, or the like. Specifically, the HDD 1400 is a recording medium that records a program for executing individual operations according to the present disclosure, which is an example of program data 1450.

The communication interface 1500 is an interface for connecting the computer 1000 to an external network 1550 (for example, the Internet). For example, the CPU 1100 receives data from other devices or transmits data generated by the CPU 1100 to other devices via the communication interface 1500.

The input/output interface 1600 is an interface for connecting between an input/output device 1650 and the computer 1000. For example, the CPU 1100 receives data from an input device such as a keyboard or a mouse via the input/output interface 1600. In addition, the CPU 1100 transmits data to an output device such as a display, a loudspeaker, or a printer via the input/output interface 1600. Furthermore, the input/output interface 1600 may function as a media interface for reading a program or the like recorded on predetermined recording media. Examples of the media include optical recording media such as a digital versatile disc (DVD) or a phase change rewritable disk (PD), a magneto-optical recording medium such as a magneto-optical disk (MO), a tape medium, a magnetic recording medium, and semiconductor memory.

For example, when the computer 1000 functions as the content generation device 100 according to the embodiment, the CPU 1100 of the computer 1000 executes the program loaded on the RAM 1200 so as to implement the functions of the control unit 130 or the like. In addition, the HDD 1400 stores a program according to the present disclosure and data in the storage unit 120. While the CPU 1100 executes program data 1450 read from the HDD 1400, the CPU 1100 may acquire these programs from another device via the external network 1550, as another example.

### <<6. Conclusion>>

The embodiments of the present disclosure have been described above. However, the technical scope of the present disclosure is not limited to the above-described embodiments, and various modifications can be made without departing from the scope of the present disclosure. Moreover, it is allowable to combine the components across different embodiments and modifications as appropriate.

For example, the control device that controls the content generation device 100 and the content providing device 200 of the present embodiment may be actualized by a dedicated computer system or a general-purpose computer system.

For example, a program for executing the above-described operations is stored in a computer-readable recording medium such as an optical disk, semiconductor memory, a magnetic tape, or a flexible disk and distributed. For example, the program is installed on a computer and the above processing is executed to achieve the composition of the control device. At this time, the control device may be a device (for example, a personal computer) outside the content generation device 100 and the content providing device 200. Furthermore, the control device may be a device (for example, the control units 130 and 230) inside the content generation device 100 and the content providing device 200.

Furthermore, the program can be stored in a disk device included in a server device on a network such as the Internet so as to be able to be downloaded to a computer, for example. Furthermore, the functions described above may be realized by using operating system (OS) and application software in cooperation. In this case, the portions other than the OS may be stored in a medium for distribution, or the portions other than the OS may be stored in a server device so as to be downloaded to a computer, for example.

Furthermore, among individual processing described in the above embodiments, all or a part of the processing described as being performed automatically may be manually performed, or the processing described as being performed manually can be performed automatically by known methods. In addition, the processing procedures, specific names, and information including various data and parameters illustrated in the above Literatures or drawings can be flexibly altered unless otherwise specified. For example, various types of information illustrated in each of the drawings are not limited to the information illustrated.

In addition, each of components of each device is provided as a functional and conceptional illustration and thus does not necessarily need to be physically constituted as illustrated. That is, the specific mode of distribution/integration of each of the devices is not limited to those illustrated in the drawings, and all or a part thereof may be functionally or physically distributed or integrated into arbitrarily chosen units in accordance with various loads and use status. This composition by distribution and integration may be performed dynamically.

Furthermore, the above-described embodiments can be appropriately combined within a range implementable without contradiction of processing. Furthermore, the order of individual steps illustrated in the sequence diagram and the flowchart of the above-described embodiment can be altered as appropriate.

Furthermore, for example, the present embodiment can be implemented as any composition constituting a device or a system, for example, a processor as a large scale integration (LSI) or the like, a module using a plurality of processors or the like, a unit using a plurality of modules or the like, and a set obtained by further adding other functions to the unit, or the like (that is, a composition of a part of the device).

In the present embodiment, a system represents a set of a plurality of components (devices, modules (parts), or the like), and whether all the components are in the same housing would not be a big issue. Therefore, a plurality of devices housed in separate housings and connected via a network, and one device in which a plurality of modules are housed in one housing, are both systems.

Furthermore, for example, the present embodiment can adopt a composition of cloud computing in which one function is cooperatively shared and processed by a plurality of devices or devices via a network.

The effects described in the embodiment of the present specification are merely examples, and thus, there may be other effects, not limited to the exemplified effects.

Note that the present technology can also have the following compositions.
(1) An information processing method comprising:
   acquiring an evaluation perspective used at a time of evaluating a motion of an evaluation target;
   determining a configuration evaluating the motion of the evaluation target based on the evaluation perspective;
   acquiring at least one of video information related to the motion and audio information; and
   generating content including the configuration and at least one of the video information and the audio information.
(2) The information processing method according to (1), wherein the evaluation perspective includes determination information related to at least one determination item for the motion and evaluation information related to an evaluation result.
(3) The information processing method according to (2), wherein the evaluation information is set in accordance with whether the at least one determination item is satisfied, and includes information related to a message to be presented to the evaluation target in accordance with the evaluation result.
(4) The information processing method according to (2) or (3), wherein the evaluation information includes predefining information predefining a presentation method in a case where the evaluation result is presented to the evaluation target.
(5) The information processing method according to any one of (2) to (4), wherein the determination information includes precondition information related to a determination precondition and passing information related to a passing condition.
(6) The information processing method according to (5), wherein the precondition information includes timing information related to a timing to perform evaluation.
(7) The information processing method according to (6), wherein the timing is one of: at any time; a timing at which the motion, as a designated motion, reaches a peak; or a timing at which the designated motion reaches a designated state.
(8) The information processing method according to c any one of (5) to (7), wherein the passing information includes at least one of: site information related to a body site; parameter information related to a motion parameter of the body site; and threshold information related to a threshold of the motion parameter.
(9) The information processing method according to (8), wherein the passing information includes one or more evaluation formulas expressed in a form of logical operation.
(10) The information processing method according to (8) or (9), further comprising
   determining the threshold information based on the video information related to the motion.
(11) The information processing method according to (10), wherein the determination of the threshold information is made based on the video information satisfying the determination item and the video information not satisfying the determination item.
(12) The information processing method according to (11), wherein
   the threshold information is determined by using a threshold determination model, and
   the threshold determination model is generated based on machine learning using the video information satisfying the determination item and the video information not satisfying the determination item.
(13) The information processing method according to any one of (1) to (12), wherein the configuration includes information related to a section in which evaluation of the motion is not performed.
(14) The information processing method according to any one of (1) to (13), wherein the evaluation perspective is generated based on at least one template predefined in preparation.
(15) The information processing method according to (14), wherein the template is predefined in preparation in accordance with the motion, the evaluation target, and a locational region.
(16) The information processing method according to any one of (1) to (15), further comprising:
   evaluating the motion included in the video information based on the configuration to verify the configuration; and
   presenting a verification result.
(17) The information processing method according to (16), wherein
   the presentation of the verification result includes
   at least one of: image information obtained by extracting an image at a timing of execution of the evaluation from the video information; information related to a determination result of at least one determination item for the motion; and evaluation information related to an evaluation result.
(18) The information processing method according to (17), wherein
   the presentation of the verification result is
   performed by superimposing, on the image information, at least one of posture information indicating a posture of the evaluation target and analysis information indicating an analysis result of the motion.
(19) The information processing method according to any one of (1) to (18), further comprising generating edited content including at least one piece of the content.
(20) The information processing method according to (19), wherein the generation of the edited content includes changing at least a part of the configuration included in the content.
(21) The information processing method according to (19) or (20), further comprising:
   acquiring user information related to a user; and
   transmitting the edited content corresponding to the user information to the user.
(22) The information processing method according to any one of (19) to (21), further comprising:
   acquiring an evaluation target video related to the motion performed by a user in accordance with the content included in the edited content; and
   evaluating the evaluation target video in accordance with the configuration.
(23) The information processing method according to any one of (1) to (22), wherein the motion is a motion related to training including at least one of rehabilitation, exercise, sports, and fitness.
(24) An information processing device comprising
   a control unit configured to:
   acquire an evaluation perspective used at a time of evaluating a motion of an evaluation target;
   determine a configuration evaluating the motion of the evaluation target based on the evaluation perspective;
   acquire at least one of video information related to the motion and audio information; and
   generate content including the configuration and at least one of the video information and the audio information.
(25) A computer-readable non-transitory storage medium storing a program, the program causing a computer to execute:
   acquiring an evaluation perspective used at a time of evaluating a motion of an evaluation target;
   determining a configuration evaluating the motion of the evaluation target based on the evaluation perspective;
   acquiring at least one of video information related to the motion and audio information; and
   generating content including the configuration and at least one of the video information and the audio information.

### Reference Signs List

10 INFORMATION PROCESSING SYSTEM
100 CONTENT GENERATION DEVICE
110, 210 COMMUNICATION UNIT
120, 220 STORAGE UNIT
130, 230 CONTROL UNIT
131 DISPLAY CONTROL UNIT
132 ACQUISITION UNIT
133 DEFINITION GENERATION UNIT
134 VERIFICATION UNIT
135 CONTENT GENERATION UNIT
136 THRESHOLD GENERATION UNIT
200 CONTENT PROVIDING DEVICE
231 PRESCRIPTION APPLICATION EXECUTION UNIT
232 DISTRIBUTION APPLICATION EXECUTION UNIT
233 CONTENT MANAGEMENT UNIT
300, 400, 500 INFORMATION PROCESSING DEVICE

## Claims

1. An information processing method comprising:
acquiring an evaluation perspective used at a time of evaluating a motion of an evaluation target;
determining a configuration evaluating the motion of the evaluation target based on the evaluation perspective;
acquiring at least one of video information related to the motion and audio information; and
generating content including the configuration and at least one of the video information and the audio information.

2. The information processing method according to claim **1,** wherein the evaluation perspective includes determination information related to at least one determination item for the motion and evaluation information related to an evaluation result.

3. The information processing method according to claim **2,** wherein the evaluation information is set in accordance with whether the at least one determination item is satisfied, and includes information related to a message to be presented to the evaluation target in accordance with the evaluation result.

4. The information processing method according to claim 2, wherein the evaluation information includes predefining information predefining a presentation method in a case where the evaluation result is presented to the evaluation target.

5. The information processing method according to claim 2, wherein the determination information includes precondition information related to a determination precondition and passing information related to a passing condition.

6. The information processing method according to claim 5, wherein the precondition information includes timing information related to a timing to perform evaluation.

7. The information processing method according to claim 6, wherein the timing is one of: at any time; a timing at which the motion, as a designated motion, reaches a peak; or a timing at which the designated motion reaches a designated state.

8. The information processing method according to claim 5, wherein the passing information includes at least one of: site information related to a body site; parameter information related to a motion parameter of the body site; and threshold information related to a threshold of the motion parameter.

9. The information processing method according to claim 8, wherein the passing information includes one or more evaluation formulas expressed in a form of logical operation.

10. The information processing method according to claim 8, further comprising
determining the threshold information based on the video information related to the motion.

11. The information processing method according to claim 10, wherein the determination of the threshold information is made based on the video information satisfying the determination item and the video information not satisfying the determination item.

12. The information processing method according to claim 11, wherein
the threshold information is determined by using a threshold determination model, and
the threshold determination model is generated based on machine learning using the video information satisfying the determination item and the video information not satisfying the determination item.

13. The information processing method according to claim 1, wherein the configuration includes information related to a section in which evaluation of the motion is not performed.

14. The information processing method according to claim 1, wherein the evaluation perspective is generated based on at least one template predefined in preparation.

15. The information processing method according to claim 14, wherein the template is predefined in preparation in accordance with the motion, the evaluation target, and a locational region.

16. The information processing method according to claim 1, further comprising:
evaluating the motion included in the video information based on the configuration to verify the configuration; and
presenting a verification result.

17. The information processing method according to claim 16, wherein
the presentation of the verification result includes
at least one of: image information obtained by extracting an image at a timing of execution of the evaluation from the video information; information related to a determination result of at least one determination item for the motion; and evaluation information related to an evaluation result.

18. The information processing method according to claim 17, wherein
the presentation of the verification result is
performed by superimposing, on the image information, at least one of posture information indicating a posture of the evaluation target and analysis information indicating an analysis result of the motion.

19. The information processing method according to claim 1, further comprising generating edited content including at least one piece of the content.

20. The information processing method according to claim 19, wherein the generation of the edited content includes changing at least a part of the configuration included in the content.

21. The information processing method according to claim 19, further comprising:
acquiring user information related to a user; and
transmitting the edited content corresponding to the user information to the user.

22. The information processing method according to claim 19, further comprising:
acquiring an evaluation target video related to the motion performed by a user in accordance with the content included in the edited content; and
evaluating the evaluation target video in accordance with the configuration.

23. The information processing method according to claim 1, wherein the motion is a motion related to training including at least one of rehabilitation, exercise, sports, and fitness.

24. An information processing device comprising
a control unit configured to:
acquire an evaluation perspective used at a time of evaluating a motion of an evaluation target;
determine a configuration evaluating the motion of the evaluation target based on the evaluation perspective;
acquire at least one of video information related to the motion and audio information; and
generate content including the configuration and at least one of the video information and the audio information.

25. A computer-readable non-transitory storage medium storing a program, the program causing a computer to execute:
acquiring an evaluation perspective used at a time of evaluating a motion of an evaluation target;
determining a configuration evaluating the motion of the evaluation target based on the evaluation perspective;
acquiring at least one of video information related to the motion and audio information; and
generating content including the configuration and at least one of the video information and the audio information.
